# EUROPEAN PATENT APPLICATION

(11) **EP 4 610 346 A1**
(43) Date of publication of application: **03.09.2025**
(21) Application number: 23882778.6
(22) Date of filing: 27.10.2023
(51) Int. Cl.: C12N 1/00, A23J 3/04, C12N 5/07, C12N 5/071, C12N 5/0775, C12P 1/00, C12P 21/00

(54) **ANIMAL CELL CULTURING METHOD**

(30) Priority: 27.10.2022 JP 2022172090; 09.03.2023 JP 2023036701; 09.08.2023 JP 2023130246
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); HIGUCHI, Takuya, Kawasaki-shi, Kanagawa 210-8681 (JP); HASEGAWA, Tatsuya, Kawasaki-shi, Kanagawa 210-8681 (JP)
(74) Representative: Hoffmann Eitle
(86) International application number: PCT/JP2023/038979
(87) International publication number: WO 2024/090577

(57) **Abstract**

A method for culturing animal cells and related techniques are provided. Animal cells are cultured in the presence of a pea-derived material and/or yeast extract.

## Description

### Technical Field

The present invention relates to a method for culturing animal cells and techniques relating thereto.

### Background Art

For culturing animal cells, media containing serum such as fetal bovine serum are widely used. However, from the viewpoints of safety, economy etc., it is desired to develop serum-free culture media that do not contain serum. For example, serum-free culture media in which serum is replaced with alternative ingredients such as growth factors have been developed.

### Summary of the Invention

### Object to be achieved by the invention

An object of the present invention is to provide a method for culturing animal cells and techniques relating thereto.

### Means for achieving the object

The inventors of the present invention found that animal cells can be cultured in the absence of serum by using a pea-derived material or yeast extract.

Thus, the present invention can be embodied, for example, as follows
[1] A composition for culturing animal cells, which contains a pea-derived material and/or yeast extract.
[2] The composition described above (specifically according to [1]), which is for improving growth and/or promoting differentiation of animal cells.
[3] The composition described above (specifically according to [1] or [2]), which is a culture medium or a culture medium additive.
[4] The composition described above (specifically according to [3]), wherein the medium is a basal medium, a feed medium, or a perfusion medium.
[5] The composition described above (specifically according to any one of [1] to [4]), wherein the pea-derived material is a pea fermentation product and/or a pea protein.
[6] The composition described above (specifically according to [5]), wherein the pea fermentation product is pea-based soy sauce (soy-free).
[7] The composition described above (specifically according to any one of [1] to [6]), wherein the animal is a mammal, bird, fish, or crustacean.
[8] The composition described above (specifically according to any one of [1] to [7]), wherein the animal is an animal for meat.
[9] The composition described above (specifically according to any one of [1] to [8]), wherein the animal is a bovine or chicken.
[10] The composition described above (specifically according to any one of [1] to [9]), wherein the cells are MyoD+ cells or PAX7+ cells.
[11] The composition described above (specifically according to any one of [1] to [10]), wherein the cells are myoblasts or satellite cells.
[12] The composition described above (specifically according to any one of [1] to [11]), which is substantially free of serum.
[13] The composition described above (specifically according to any one of [1] to [12]), which is substantially free of animal-derived albumin.
[14] A method for producing a product, which comprises
   the step of culturing animal cells in the presence of a pea-derived material and/or yeast extract.
[15] The method described above (specifically according to [14]), wherein the product is cultured meat.
[16] A method for culturing animal cells, which comprises
   the step of culturing the animal cells in the presence of a pea-derived material and/or yeast extract.
[17] A method for improving growth of animal cells, which comprises
   the step of culturing the animal cells in the presence of a pea-derived material and/or yeast extract.
[18] A method for promoting differentiation of animal cells, which comprises
   the step of culturing the animal cells in the presence of a pea-derived material and/or yeast extract.
[19] The method described above (specifically according to any one of [14] to [18]), wherein the pea-derived material is a pea fermentation product and/or a pea protein.
[20] The method described above (specifically according to [19]), wherein the pea fermentation product is pea-based soy sauce (soy-free).
[21] The method described above (specifically according to [19] or [20]), wherein concentration of the pea fermentation product in a medium during the culture is 0.1 to 100 mg/mL.
[22] The method described above (specifically according to any one of [19] to [21]), wherein concentration of the pea protein in a medium during the culture is 0.05 to 50 mg/mL.
[23] The method described above (specifically according to any one of [14] to [22]), wherein the animal is a mammal, bird, fish, or crustacean.
[24] The method described above (specifically according to any one of [14] to [23]), wherein the animal is an animal for meat.
[25] The method described above (specifically according to any one of [14] to [24]), wherein the animal is a bovine or chicken.
[26] The method described above (specifically according to any one of [14] to [25]), wherein the cells are MyoD+ cells or PAX7+ cells.
[27] The method described above (specifically according to any one of [14] to [26]), wherein the cells are myoblasts or satellite cells.
[28] The method described above (specifically according to any one of [14] to [27]), wherein the culture is performed in a medium substantially free of serum.
[29] The method described above (specifically according to any one of [14] to [28]), wherein the culture is performed in a medium substantially free of animal-derived albumin.

### Brief Description of the Drawings

[Fig. 1] A graph showing proliferation of bovine muscle stem cells in a serum-free medium in the presence of pea-based soy sauce (soy-free).
[Fig. 2] A graph showing proliferation rate of bovine muscle stem cells in a serum-free medium in the presence of pea-based soy sauce (soy-free).
[Fig. 3] A graph showing proliferation of bovine muscle stem cells in a serum-free medium in the presence of pea-based soy sauce (soy-free), soy sauce, or soy protein acid hydrolysate.
[Fig. 4] A graph showing proliferation rate of bovine muscle stem cells in a serum-free medium in the presence of pea-based soy sauce (soy-free), soy sauce, or soy protein acid hydrolysate.
[Fig. 5] A graph showing proliferation of bovine muscle stem cells in a serum-free medium in the presence of pea-based soy sauce (soy-free) or pea protein hydrolysate.
[Fig. 6] A graph showing proliferation rate of bovine muscle stem cells in a serum-free medium in the presence of pea-based soy sauce (soy-free) or pea protein hydrolysate.
[Fig. 7] A graph showing proliferation of bovine muscle stem cells in a serum-free medium in the presence of plant proteins.
[Fig. 8] A graph showing proliferation rate of bovine muscle stem cells in a serum-free medium in the presence of plant proteins.
[Fig. 9] A graph showing proliferation rate of bovine muscle stem cells in a serum-free medium in the presence of soy protein.
[Fig. 10] A graph showing proliferation of bovine muscle stem cells in a serum-free medium in the presence of pea protein.
[Fig. 11] A graph showing proliferation rate of bovine muscle stem cells in a serum-free medium in the presence of pea protein.
[Fig. 12] A graph showing proliferation of bovine muscle stem cells in a serum-free medium in the presence of pea-based soy sauce (soy-free) and/or pea protein.
[Fig. 13] A graph showing proliferation rate of bovine muscle stem cells in a serum-free medium in the presence of pea-based soy sauce (soy-free) and/or pea protein.
[Fig. 14] A graph showing proliferation of human muscle stem cells in a serum-free medium in the presence of pea-based soy sauce (soy-free) or yeast extract.
[Fig. 15] A graph showing differentiation of chicken muscle stem cells in a serum-free medium in the presence of pea-based soy sauce (soy-free), pea protein, soy sauce, soy protein acid hydrolysate, or yeast extract.
[Fig. 16] A graph showing proliferation of chicken muscle stem cells in a serum-free medium in the presence of pea-based soy sauce (soy-free), pea protein, soy sauce, soy protein acid hydrolysate, or yeast extract.

### Modes for Carrying out the Invention

### <1> Active ingredient

### <1-1> Active ingredient

In the present invention, a pea-derived material and/or yeast extract is used as an active ingredient.

The pea-derived material and/or yeast extract is also collectively referred to as "active ingredient".

The active ingredient can be used to culture animal cells. Specifically, animal cells can be cultured in the presence of the active ingredient.

By using the active ingredient, specifically by culturing animal cells in the presence of the active ingredient, growth of the animal cells may be improved, i.e., an effect of improving growth of animal cells may be obtained. This effect is also referred to as the "growth-improving effect". In other words, the active ingredient may have a function of improving growth of animal cells. This function is also referred to as the "growth-improving function". That is, culturing animal cells in the presence of the active ingredient may improve growth of the animal cells compared with culturing animal cells in the absence of the active ingredient. Examples of culturing animal cells in the absence of the active ingredient includes culturing animal cells under the same conditions as those used for culturing animal cells in the presence of the active ingredient, except that the active ingredient is not utilized. The growth-improving effect may be obtained, for example, when animal cells are cultured under conditions in which serum is substantially absent (i.e., animal cells are cultured in a medium that is substantially free of serum). The growth-improving effect may be obtained, for example, when animal cells are cultured under conditions in which animal-derived albumin is substantially absent (i.e., animal cells are cultured in a medium that is substantially free of animal-derived albumin). The growth-improving effect may be obtained, for example, when animal cells are cultured under conditions in which serum and animal-derived albumin are substantially absent (i.e., animal cells are cultured in a medium that is substantially free of serum and animal-derived albumin). The growth-improving effect may be obtained, for example, when the animal cells are MyoD+ cells (i.e., cells expressing MyoD). MyoD+ cells may be, for example, myoblasts. Examples of the improvement of growth include an increase in proliferation rate. The growth-improving effect can be confirmed by measuring and comparing growths (e.g., proliferation rates) of animal cells in the presence and absence of the active ingredient.

By utilizing the active ingredient, specifically by culturing animal cells in the presence of the active ingredient, differentiation of the animal cells may be promoted, i.e., an effect of promoting differentiation of animal cells may be obtained. This effect is also referred to as the "differentiation-promoting effect. In other words, the active ingredient may have a function of promoting differentiation of animal cells. This function is also referred to as the "differentiation-promoting function". That is, culturing animal cells in the presence of the active ingredient may promote differentiation of the animal cells compared with culturing animal cells in the absence of the active ingredient. Examples of culturing animal cells in the absence of the active ingredient includes culturing animal cells under the same conditions as those used for culturing the animal cells in the presence of the active ingredient, except that the active ingredient is not utilized. The differentiation-promoting effect may be obtained, for example, when animal cells are cultured under conditions in which serum is substantially absent (i.e., animal cells are cultured in a medium that is substantially free of serum). The differentiation-promoting effect may be obtained, for example, when animal cells are cultured under conditions in which animal-derived albumin is substantially absent (i.e., animal cells are cultured in a medium that is substantially free of animal-derived albumin). The differentiation-promoting effect may be obtained, for example, when animal cells are cultured under conditions in which serum and animal-derived albumin are substantially absent (i.e., animal cells are cultured in a medium that is substantially free of serum and animal-derived albumin). The differentiation-promoting effect may be obtained, for example, when the animal cells are PAX7+ cells (i.e., cells expressing PAX7). PAX7+ cells may be, for example, satellite cells. Satellite cells may be activated. The differentiation-promoting effect may be obtained, for example, when animal cells are cells of a bird such as chicken. The differentiation-promoting effect may specifically be obtained, for example, when the animal cells are PAX7+ cells of a bird such as chicken. Examples of the differentiation may include muscle differentiation. Examples of promotion of differentiation include an increase in differentiation rate and an increase in ratio of differentiated cells. The differentiation-promoting effect can be confirmed by measuring and comparing differentiations of animal cells (e.g., differentiation rates or ratios of differentiated cells) observed in the presence and absence of the active ingredient. Differentiation of animal cells can be measured by, for example, using expression of a protein that can be an indicator of differentiation as an indicator. For example, in muscle differentiation, Sprouty1, Pax7, MyoD, myogenin, and myosin heavy chain (MHC) can be expressed in sequence as differentiation progresses (Manuel Schmidt et al. Adult stem cells at work: regenerating skeletal muscle. Cell Mol Life Sci. 2019 Jul;76(13):2559-2570).

The use of the active ingredient, specifically culturing animal cells in the presence of the active ingredient, may provide the growth-improving effect and differentiation-promoting effect.

In one embodiment, a product may be produced by culturing animal cells. Examples of the product include cultured meat and a target substance. The target substance is not particularly limited as long as it can be produced by animal cells. Examples of the target substance include proteins and viruses. The product may be, in particular, cultured meat. When a product is produced by culturing animal cells, the production of the product may be improved by using the active ingredient. That is, culturing animal cells in the presence of the active ingredient may improve the production of the product compared with culturing animal cells in the absence of the active ingredient. For example, the production of the product may be improved by improving growth and/or promoting differentiation of the animal cells through the use of the active ingredient. That is, one aspect of the growth-improving effect or the differentiation-promoting effect may be an effect of improving production of a product. The effect of improving production of a product may be obtained, for example, when animal cells are cultured under conditions in which serum is substantially absent (i.e., the animal cells are cultured in a medium that is substantially free of serum). The effect of improving production of a product may be obtained, for example, when animal cells are cultured under conditions in which animal-derived albumin is substantially absent (i.e., the animal cells are cultured in a medium that is substantially free of animal-derived albumin). The effect of improving production of a product may be obtained, for example, when animal cells are cultured under conditions in which serum and animal-derived albumin are substantially absent (i.e., animal cells are cultured in a medium that is substantially free of serum and animal-derived albumin). Examples of the improvement of production include increases in production amount and production rate.

### <1-1-1> Pea-derived material

The term "pea-derived material" means a material derived from peas.

The type of the pea-derived material is not particularly limited as long as it has the desired function (e.g., growth-improving function or differentiation-promoting function). Examples of the pea-derived material include processed products of peas. Examples of the pea-derived material (e.g., processed product of peas) include pea fermentation products and pea proteins. As the pea-derived material, one type of material may be used, or two or more types of materials may be used in combination.

The type of peas is not particularly limited as long as the pea-derived material has the desired function (e.g., growth-improving function or differentiation-promoting function). Examples of peas include seed peas and podded peas. The seed peas include mature peas such as green peas, red peas, and yellow peas, as well as immature peas such as immature green peas. As the peas, especially seed peas can be mentioned. As the peas, more particularly, mature peas can be mentioned. As the peas, one type of peas may be used, or two or more types of peas may be used in combination. That is, the pea-derived material may be derived from one type of peas, or may be derived from two or more types of peas. That is, as the pea-derived material, a material derived from one type of peas may be used, or materials derived from two or more types of peas may be used in combination.

### <1-1-1> Pea fermentation product

The term "pea fermentation product" means a product obtained by fermenting peas with microorganisms. "Fermentation" referred to here may mean to be used for culture as a medium ingredient. That is, "to ferment peas with microorganisms" may mean to culture the microorganisms in a medium containing peas. That is, the "pea fermentation product" may specifically mean a product obtained by culturing microorganisms in a medium containing peas. As the pea fermentation product, one type of fermentation product may be used, or two or more types of fermentation products may be used in combination.

The type of peas from which the pea fermentation product is derived is not particularly limited as long as the pea fermentation product can be produced. The peas are as described above. The pea fermentation product may be derived from one type of peas or derived from two or more types of peas. That is, as the pea fermentation product, a fermentation product derived from one type of peas may be used, or fermentation products derived from two or more types of peas may be used in combination.

Examples of the pea fermentation product include pea-based soy sauce (soy-free). The term "pea-based soy sauce (soy-free)" may refer to a soy sauce-like seasoning producing by using peas as a raw material. The term "soy sauce-like seasoning" may refer to a liquid seasoning or processed product thereof that can be used in the same way as "soy sauce" defined in the Japanese Agricultural Standards. Examples of the processed product include dried products (e.g., dried powder). Examples of the pea-based soy sauce (soy-free) include one produced by the method described in Japanese Patent Publication (Kokai) No. 2014-110771.

An exemplary method for producing a pea fermentation product will be explained below. That is, the pea fermentation product may be one produced by the following production method.

The pea fermentation product can be produced by fermenting peas with microorganisms (specifically, culturing microorganisms in a medium containing peas).

That is, the microorganisms may be first cultured in a medium containing peas. That is, the method for producing a pea fermentation product may comprise the step of culturing a microorganism in a medium containing peas. This step is also referred to as "culturing step". The culturing step may specifically be a step of culturing a microorganism in a medium containing peas to obtain a culture product. The culture product is also referred to as "fermentation product".

The type of the microorganism is not particularly limited as long as it can produce a pea fermentation product. Examples of the microorganism include bacteria and fungi.

Examples of bacteria include lactic acid bacteria.

Examples of lactic acid bacteria include *Tetragenococcus* bacteria such as *Tetragenococcus halophilus, Bifidobacterium* bacteria such as *Bifidobacterium longum, Bifidobacterium breve, Bifidobacterium bifidum, Bifidobacterium adolescentis, Bifidobacterium angulatum, Bifidobacterium dentium, Bifidobacterium pseudocatenulatum, Bifidobacterium animalis, Bifidobacterium pseudolongum,* and *Bifidobacterium thermophilum,* and *Lactobacillus* bacteria such as *Lactobacillus mali, Lactobacillus hilgardii, Lactobacillus brevis, Lactobacillus delbrueckii, Lactobacillus casei, Lactobacillus paracasei, Lactobacillus gasseri,* and *Lactobacillus acidophilus.* As the lactic acid bacteria, *Tetragenococcus* bacteria can be especially mentioned. As the lactic acid bacteria, *Tetragenococcus halophilus* can be more particularly mentioned. The lactic acid bacteria may be, for example, salt-tolerant lactic acid bacteria. The term "salt-tolerant lactic acid bacteria" may mean, for example, lactic acid bacteria that can proliferate in a medium containing 8% (w/w) of sodium chloride. For example, *Tetragenococcus halophilus* can be an example of the salt-tolerant lactic acid bacteria. As the lactic acid bacteria, for example, lactic acid bacteria commonly used in the production of soy sauce may be selected.

Examples of the fungi include yeasts and filamentous fungi.

The yeasts may be a budding yeast or fission yeast. The yeasts may be a monoploid yeast, diploid yeast or yeast of higher ploidy. Examples of the yeasts include *Zygosaccharomyces* yeasts such as *Zygosaccharomyces rouxii* and *Zygosaccharomyces sapae, Saccharomyces* yeasts such as *Saccharomyces cerevisiae, Pichia* (also called *Wickerhamomyces*) yeasts such as *Pichia farinosa, Pichia ciferrii, Pichia sydowiorum,* and *Pichia pastoris, Candida* yeasts such as *Candida versatilis, Candida etschellsii,* and *Candida utilis, Hansenula* yeasts such as *Hansenula polymorpha,* and *Schizosaccharomyces* yeasts such as *Schizosaccharomyces pombe.* As the yeasts, *Zygosaccharomyces rouxii, Zygosaccharomyces sapae, Pichia farinosa, Candida versatilis, Candida etschellsii, Saccharomyces cerevisiae,* and *Schizosaccharomyces pombe* can be especially mentioned. As the yeasts, yeasts of the genus *Zygosaccharomyces* can also be especially mentioned. As the yeasts, *Zygosaccharomyces rouxii* can be more especially mentioned. Examples of *Zygosaccharomyces rouxii* include the *Zygosaccharomyces rouxii* NBRC 1130 strain (ATCC 56077). Examples of *Saccharomyces cerevisiae* includes the *Saccharomyces cerevisiae* NBRC 10217 strain (ATCC 18824), BY4742 strain (ATCC 201389), BY4743 strain (ATCC 201390), and S288C strain (ATCC 26108). Examples of *Schizosaccharomyces pombe* include the *Schizosaccharomyces pombe* NBRC 1628 strain. The yeasts may be, for example, a salt-tolerant yeast. The term "salt-tolerant yeast" may mean, for example, a yeast that can proliferate in a medium containing 8% (w/w) of sodium chloride. For example, *Zygosaccharomyces rouxii, Zygosaccharomyces sapae, Pichia farinosa, Candida versatilis,* and *Candida etschellsii* can all be examples of salt-tolerant yeast. As the yeasts, for example, yeasts commonly used in the production of soy sauce may be selected.

Examples of the filamentous fungi include *Aspergillus* fungi such as *Aspergillus oryzae, Aspergillus sojae, Aspergillus niger, Aspergillus tamarii, Aspergillus kawachii, Aspergillus awamori,* and *Aspergillus saitoi.* As the filamentous fungi, *Aspergillus oryzae* and *Aspergillus sojae* can be especially mentioned. As the filamentous fungi, for example, filamentous fungi commonly used in the production of soy sauce (specifically, production of koji) may be selected.

These strains can be obtained from, for example, the American Type Culture Collection (ATCC, Address: 10801 University Boulevard Manassas, VA 20110, United States of America). That is, a registration number corresponding to each strain is assigned, and this registration number can be used to order each strain (see http://www.atcc.org/). The registration numbers for the respective strains are listed in the catalog of the American Type Culture Collection. These strains can also be obtained from, for example, the NITE Biological Resource Center (NBRC). These strains can also be obtained from, for example, the depositories where the strains are deposited.

As the microorganism, one type of microorganisms may be used, or two or more types of microorganisms may be used in combination. As the microorganism, for example, filamentous fungi, yeasts, and lactic acid bacteria may be used in combination. When two or more types of microorganisms are used in combination, they may or may not be cultured simultaneously. For example, filamentous fungi may be cultured first, followed by culturing yeasts and lactic acid bacteria. When a certain microorganism A is first cultured, and then a certain microorganism B is cultured, "peas" used in the culture of the microorganism B may mean peas remaining after the culture of the microorganism A (i.e., peas fermented by the microorganism A).

The culture conditions are not particularly limited as long as a pea fermentation product can be produced. The culture can be performed, for example, under the conventional conditions used for culturing microorganisms, except that a medium containing peas is used. The culture may be performed, for example, as solid culture (i.e., on a solid medium) or liquid culture (i.e., in a liquid medium). The culture may be performed, in particular, as liquid culture (i.e. in a liquid medium).

The medium used for the culture is not particularly limited as long as it contains peas and allows production of a pea fermentation product. A medium containing, in addition to peas, for example, a carbon source, nitrogen source, phosphate source, sulfur source, and other ingredients selected from various organic and inorganic ingredients as needed can be used. The medium may be peas themselves. That is, the microorganism may be inoculated directly on peas and cultured. The types and concentrations of the medium ingredients can be appropriately set by those skilled in the art. For specific medium ingredient composition, for example, conventional medium ingredient compositions used for culturing microorganisms can be referred to. The medium may contain one type of peas or two or more types of peas in combination.

The form of peas for use in the culture is not particularly limited as long as a pea fermentation product can be produced. The peas may be used in the culture, for example, after separated from the pods, or without separation from the pods. For example, fresh peas (specifically, fresh edible parts) may be used for the culture, either as they are or after being processed as appropriate. Examples of such processing include cutting, crushing, straining, pressing, fractionation, dilution, concentration, drying, and heating. These processes may be performed individually or in an appropriate combination.

Examples of the carbon sources include sugars. Specific examples of sugars include glucose, fructose, galactose, xylose, arabinose, sucrose, lactose, cellobiose, blackstrap molasses, starch hydrolysate, and biomass hydrolysate. As the carbon source, one type of carbon source may be used, or two or more types of carbon sources may be used in combination.

Specific examples of the nitrogen sources include ammonium salts such as ammonium sulfate, ammonium chloride, and ammonium phosphate, organic nitrogen source such as peptone, casein peptone, soy peptone, potato peptone, wheat peptone, casamino acid, yeast extract, malt extract, meat extract, and corn steep liquor, ammonia, and urea. As the nitrogen source, one type of nitrogen source may be used, or two or more types of nitrogen sources may be used in combination.

Specific examples of the phosphoric acid sources include phosphates such as potassium dihydrogenphosphate and dipotassium hydrogenphosphate, and phosphoric acid polymers such as pyrophosphoric acid. One type of phosphoric acid source may be used, or two or more types of phosphoric acid sources may be used in combination.

Specific examples of the sulfur sources include inorganic sulfur compounds such as sulfates, thiosulfates, and sulfites, and sulfur-containing amino acids such as cysteine, cystine, and glutathione. One type of sulfur source may be used, or two or more types of sulfur sources may be used in combination.

Specific examples of the other various organic and inorganic ingredients include, for example, inorganic salts such as sodium chloride and potassium chloride; trace metals such as iron, manganese, magnesium, and calcium; vitamins such as vitamin B1, vitamin B2, vitamin B6, nicotinic acid, nicotinic acid amide, and vitamin B12; amino acids; nucleic acids; organic ingredients containing the foregoing ingredients such as peptone, casamino acid, yeast extract, malt extract, meat extract, and corn steep liquor. As such various other organic and inorganic ingredients, a single type of ingredient may be used, or two or more types of ingredients may be used in combination.

The medium may contain, for example, at least sodium chloride. That is, the medium may contain, for example, peas and sodium chloride.

The culture may be performed, for example, under aerobic conditions. In the case of aerobic conditions, the dissolved oxygen concentration in the culture medium may be controlled to be, for example, 5 to 50%, preferably 10 to 20%, in terms of a ratio to the saturated dissolved oxygen concentration taken as 100%. The culture under aerobic conditions may specifically be performed, for example, as static culture, aerated culture, shaking culture, stirred culture, or a combination thereof. The culture temperature may be, for example, 25 to 45°C, or 25 to 35°C, preferably 27 to 33°C, more preferably 28 to 32°C. The culture period may be, for example, 10 hours to 6 months, 10 hours to 2 months, 10 hours to 1 month, or 10 hours to 200 hours, preferably 15 hours to 120 hours. The pH of the medium may be, for example, 3 to 10, preferably 3 to 8. During the culture, pH of the medium may be adjusted as needed. For pH adjustment, inorganic or organic acidic or alkaline substances, e.g., ammonia gas etc. may be used. The culture may be performed as, for example, batch culture, fed-batch culture, continuous culture, or a combination thereof. The culture may be divided into pre-culture and main culture. For example, the pre-culture may be performed on a solid medium such as agar medium, and the main culture may be conducted in a liquid medium. When the culture is divided into and performed as pre-culture and main culture, the expression that "the medium contains a certain ingredient" means that the medium contains the ingredient at least during the main culture. That is, for example, peas are contained in the culture medium during at least the main culture. The culture may be continued, for example, until a culture product having a desired function (e.g., growth-improving function or differentiation-promoting function) is obtained.

The culture can be performed, for example, in the manner described in Japanese Patent Publication (Kokai) No. 2014-110771. That is, for example, filamentous fungi can first be inoculated on peas that have been pretreated as appropriate and cultured to produce pea koji. The culture for producing pea koji may be performed, for example, at 25 to 45°C and 85 to 95% humidity for 12 to 240 hours. The pea koji can then be mixed with an aqueous sodium chloride solution, yeast and lactic acid bacteria can be inoculated, and culture (specifically, ripening) can be performed to produce a fermentation product. The ripening may be performed, for example, at 15 to 35°C for 3 to 6 months with stirring as appropriate. The ripening may also be performed, for example, at 35 to 55°C for 1 day to 2 months with stirring as appropriate.

The amounts of the medium ingredients such as peas and sodium chloride in the medium are not particularly limited as long as a pea fermentation product can be produced.

The content of peas in the medium may be, for example, 0.5% (w/w) or higher, 1% (w/w) or higher, 2% (w/w) or higher, 3% (w/w) or higher, 4% (w/w) or higher, 5% (w/w) or higher, 7% (w/w) or higher, 10% (w/w) or higher, 15% (w/w) or higher, 20% (w/w) or higher, 25% (w/w) or higher, 30% (w/w) or higher, 40% (w/w) or higher, 50% (w/w) or higher, or 70% (w/w) or higher, and may be 100% (w/w) or lower, 70% (w/w) or lower, 50% (w/w) or lower, 40% (w/w) or lower, 30% (w/w) or lower, 25% (w/w) or lower, 20% (w/w) or lower, 15% (w/w) or lower, 10% (w/w) or lower, 7% (w/w) or lower, 5% (w/w) or lower, 4% (w/w) or lower, 3% (w/w) or lower, 2% (w/w) or lower, or 1% (w/w) or lower, or may be any non-contradictory combination of these minimum and maximum contents. Specifically, the content of peas in the medium may be, for example, 0.5 to 1% (w/w), 1 to 2% (w/w), 2 to 3% (w/w), 3 to 4% (w/w), 4 to 5% (w/w), 5 to 7% (w/w), 7 to 10% (w/w), 10 to 15% (w/w), 15 to 20% (w/w), 20 to 25% (w /w), 25 to 30% (w/w), 30 to 40% (w/w), 40 to 50% (w/w), 50 to 70% (w/w), or 70 to 100% (w/w). The content of peas in the medium may specifically be, for example, 0.5 to 100% (w/w), 0.5 to 50% (w/w), 1 to 30% (w/w), or 2 to 10% (w/w).

The content of sodium chloride in the medium may be, for example, 0% (w/w) or higher, 0.1% (w/w) or higher, 0.2% (w/w) or higher, 0.5% (w/w) or higher, 1% (w/w) or higher, 2% (w/w) or higher, 3% (w/w) or higher, 4% (w/w) or higher, 5% (w/w) or higher, 6% (w/w) or higher, 7% (w/w) or higher, 8% (w/w) or higher, 9% (w/w) or higher, 10% (w/w) or higher, 12% (w/w) or higher, or 15% (w/w) or higher, and may be 20% (w/w) or lower, 15% (w/w) or lower, 12% (w/w) or lower, 10% (w/w) or lower, 9% (w/w) or lower, 8% (w/w) or lower, 7% (w/w) or lower, 6% (w/w) or lower, 5% (w/w) or lower, 4% (w/w) or lower, 3% (w/w) or lower, 2% (w/w) or lower, 1% (w/w) or lower, 0.5% (w/w) or lower, 0.2% (w/w) or lower, or 0.1% (w/w) or lower, or may any non-contradictory combination of these minimum and maximum contents. The content of sodium chloride in the medium may specifically be, for example, 0 to 0.1% (w/w), 0.1 to 0.2% (w/w), 0.2 to 0.5% (w/w), 0.5 to 1% (w/w), 1 to 2% (w/w), 2 to 3% (w/w), 3 to 4% (w/w), 4 to 5% (w/w), 5 to 6% (w/w), 6 'to 7% (w/w), 7 to 8% (w/w), 8 to 9% (w/w), 9 to 10% (w/w), 10 to 12% (w/w), 12 to 15% (w/w), or 15 to 20% (w/w). The content of sodium chloride in the medium may specifically be, for example, 0 to 20% (w/w), 0.1 to 20% (w/w), 1 to 20% (w/w), 5 to 20% (w/w), 5 to 15% (w/w), or 5 to 12% (w/w). For the content of sodium chloride in the medium, the expression "0% (w/w) or higher" means the case where the culture medium does not contain sodium chloride (i.e., 0% (w/w)) and the case where the culture medium contains sodium chloride (i.e., higher than 0% (w/w)). Therefore, for example, the expression that "the content of sodium chloride in the medium is 0 to 20% (w/w)" means that the medium contains no sodium chloride or the medium contains sodium chloride at a content of 20% (w/w) or lower (specifically, at a content higher than 0% (w/w) and not higher than 20% (w/w)).

Any of the medium ingredients such as peas and sodium chloride may be contained in the medium during the entire period of the culture, or may be contained in the medium during only a part of the culture period. That is, the expression that "the culture is performed in a medium containing a certain ingredient" means that it is sufficient if the ingredient is contained in the medium during at least a part of the culture period, and it is not necessary that the ingredient be contained in the medium during the entire period of the culture. Any of the medium ingredients such as peas and sodium chloride may be, for example, contained in the medium at the start of the culture, or may be supplied to the medium after the start of the culture. Further, any of the medium ingredients such as peas and sodium chloride may be, for example, contained in the medium at the start of culture and further supplied to the medium after the start of the culture (e.g., after consumption of the ingredients).

Any of the medium ingredients such as peas and sodium chloride may be, for example, contained in the medium at the concentration exemplified above during the entire period of the culture, or may be contained in the medium at the concentration exemplified above during only a part of the culture period. That is, the expression that "the culture is performed in a medium containing a certain ingredient at a certain concentration" means that it is sufficient if the concentration of the ingredient in the medium is within the said concentration range during at least a part of the culture period, and it is not necessary that the concentration of the ingredient in the medium be the said concentration range during the entire period of the culture. Any of the medium ingredients such as peas and sodium chloride may be, for example, contained in the medium at the concentration exemplified above at the start of the culture, or may be supplied to the medium to reach the concentration exemplified above after the start of the culture. Further, any of the medium ingredients such as peas and sodium chloride may be, for example, contained in the medium at the concentration exemplified above at the start of culture and further supplied to the medium to reach the concentration exemplified above after the start of culture (e.g., after consumption of the ingredient).

The length of the "part of the culture period" is not particularly limited as long as a pea fermentation product can be produced. The length of the "part of the culture period" can be appropriately set according to various conditions, such as the type of medium ingredients, type of microorganisms, and length of the culture period. The "part of the culture period" may be, for example, 10% or more, 20% or more, 30% or more, 40% or more, 50% or more, 60% or more, 70% or more, 80% or more, 90% or more, 95% or more, 97% or more, or 99% or more of the entire culture period. The "part of the culture period" may be a period of, for example, 10 hours or longer, 20 hours or longer, 40 hours or longer, 60 hours or longer, 80 hours or longer, 100 hours or longer, 120 hours or longer, or 150 hours or longer. The "entire culture period" means the entire period of the main culture if the culture is divided into and performed as seed culture (pre-culture) and main culture.

By culturing the microorganisms as described above, a culture product (i.e., pea fermentation product) can be obtained.

The culture may be used as the pea fermentation product as it is or after being subjected to an appropriate treatment. That is, the method for producing a pea fermentation product may comprise the step of subjecting the culture to a treatment. For example, the culture may be used for the production of the composition of the present invention as the pea fermentation product, either as it is or after being subjected to an appropriate treatment. Further, for example, the culture may be used in the method of the present invention as the pea fermentation product, either as it is or after being subjected to an appropriate treatment. The treatment is not particularly limited as long as the purpose of the present invention can be achieved (e.g., growth-improving effect or differentiation-promoting effect can be obtained). Examples of the treatment include fractionation, dilution, concentration, drying, heating, and sterilization. For example, in the case of fractionation, a fraction having the desired function (e.g., growth-improving function or differentiation-promoting function) can be collected. The fraction collected by fractionation may specifically be a fraction containing the ingredient having the desired function (e.g., growth-improving function or differentiation-promoting function). The form of the pea fermentation product is not particularly limited. The pea fermentation product may be in any form, for example, powder, flake, paste, liquid, etc. That is, the culture may be processed into a desired form. Namely, the method for producing a pea fermentation product may comprise the step of processing the pea fermentation product into a desired form. For example, the culture may be dried, powdered, and used as the pea fermentation product. The method for producing a pea fermentation product may comprise, for example, the step of drying and powdering the pea fermentation product. Examples of the means for drying include freeze drying, spray drying, and drum drying. The pea fermentation product may be blended with additives such as excipients.

### <1-1-1-2> Pea protein

The term "pea protein" means a protein derived from peas. The "pea protein" is not limited to a full-length pea protein, but may also include a fragment (i.e., partial protein) thereof. Examples of the fragment of full-length pea protein include hydrolysates of the full-length pea protein. That is, for example, the pea protein may or may not be hydrolyzed. In one embodiment, the pea protein may not be hydrolyzed. As the pea protein, a single type of protein may be used, or two or more types of proteins may be used in combination.

The type of pea from which the pea protein is derived is not particularly limited as long as the pea protein has the desired function (e.g., growth-improving function or differentiation-promoting function). Peas are as described above. The pea protein may be derived from one type of pea or two or more types of peas. That is, as the pea protein, a protein derived from one type of pea may be used, or proteins derived from two or more types of peas may be used in combination.

As the pea protein, a commercially available product may be used, or a product obtained by producing it as appropriate may be used. The method for producing the pea protein is not particularly limited. The pea protein can be produced by, for example, extraction from peas. The pea protein may or may not be purified to a desired degree. That is, as the pea protein, a purified product may be used, or a pea protein-containing material may be used. Examples of the pea protein-containing material include crude extracts of pea protein (i.e., proteins crudely extracted from peas). Examples of the pea protein-containing material also include crushed peas (e.g., pea powder). Crushed peas (e.g., pea powder) can be produced by crushing peas. Peas may be, for example, crushed after separation from pods or without separation from pods. The pea protein content in the pea protein-containing material may be, for example, 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher.

The amount of the pea protein (e.g., content (concentration) or amount used) shall be calculated on the basis of the amount of the pea protein itself contained in the material, if a pea protein-containing material is used. The content of the pea protein in an object (e.g., material or composition) can be determined by, for example, a known protein quantification method such as the Kjeldahl method.

### <1-1-2> Yeast extract

The term "yeast extract" may mean an extract of yeast. As the yeast extract, one type of yeast extract may be used, or two or more types of yeast extracts may be used in combination.

The type of yeast for the yeast extract is not particularly limited as long as the yeast extract has the desired function (e.g., growth-improving function or differentiation-promoting function). Examples of the yeast for the yeast extract include the yeasts exemplified for the method for producing a pea fermentation product. Examples of the yeast for the yeast extract include, in particular, yeasts of the genus *Saccharomyces* such as *Saccharomyces cerevisiae,* yeasts of the genus *Schizosaccharomyces* such as *Schizosaccharomyces pombe,* and yeasts of the genus *Candida* such as *Candida utilis.* As the yeast for the yeast extract, yeasts of the genus *Saccharomyces* and yeasts of the genus *Candida* can be more particularly mentioned. As the yeast for the yeast extract, one type of yeast may be used or two or more types of yeasts may be used in combination.

As the yeast extract, a commercially available product may be used, or a product obtained by producing it as appropriate may be used. The method for producing the yeast extract is not particularly limited. For example, the yeast extract can be produced from yeast cells by a known method. Examples of the method for producing yeast extract include autolysis, enzymolysis, acidolysis, alkaline degradation, physical disruption, and freeze-thaw methods.

The form of the yeast extract is not particularly limited. The yeast extract may be in any form, for example, powder, flake, paste, liquid, etc.

### <1-2> Other ingredients

The active ingredient may be used in combination with, for example, another or other ingredients. Examples of such other ingredients include lecithin. That is, the active ingredient may be used in combination with, for example, lecithin. The other ingredients (e.g., lecithin) may have, for example, a growth-improving function and/or differentiation-promoting function. For the growth-improving function and differentiation-promoting function of the other ingredients, the descriptions for the growth-improving function and differentiation-promoting function of the active ingredient may be applied, respectively.

The term "lecithin" may collectively refer to any material containing a phospholipid. Lecithin may contain a phospholipid as a main ingredient. The content of phospholipid in lecithin may be, for example, 50% (w/w) or higher, 70% (w/w) or higher, 90% (w/w) or higher, or 95% (w/w) or higher. The type of lecithin is not particularly limited. For example, a lecithin having a desired function (e.g., growth-improving function or differentiation-promoting function) may be selected. Examples of lecithin include plant lecithins, egg yolk lecithin, and derivatives thereof. Examples of plant lecithins include soy lecithin, oilseed rape lecithin, and sunflower lecithin. Examples of the derivatives include fractionated lecithin, enzyme-treated lecithin, and enzyme-degraded lecithin. As lecithin, soy lecithin can be especially mentioned. As lecithin, one type of lecithin may be used, or two or more types of lecithins may be used in combination.

As lecithin, a commercially available product may be used, or a product obtained by producing it as appropriate may be used. The method for producing lecithin is not particularly limited. Lecithin can be produced by, for example, separation from a lecithin-containing raw material. Specifically, soy lecithin can be produced by, for example, separation from soybean oil.

### <2> Composition of the present invention

The composition of the present invention is a composition containing the active ingredient (i.e., pea-derived material and/or yeast extract).

The composition of the present invention may be used for, for example, culturing animal cells. That is, the composition of the present invention may be a composition for culturing animal cells. The composition of the present invention can be used for, for example, culturing animal cells in the manner described for the method of the present invention explained later.

The composition of the present invention (specifically, a composition for culturing animal cells) may be, for example, a culture medium. The medium may be a basal medium, feed medium, or perfusion medium. The term "basal medium" may mean a medium used at the start of the culture. The basal medium is also referred to as "starting medium". The "feed medium" may mean a medium supplied to the culture system after the start of the culture in fed-batch culture. The "perfusion medium" may mean a medium supplied to the culture system after the start of the culture in continuous culture (which is not limited to perfusion culture).

The composition of the present invention (specifically, a composition for culturing animal cells) may be, for example, a medium additive. The term "medium additive" may mean a composition that is used by adding it to a culture medium. Examples of the medium to which the medium additive is added include a basal medium, feed medium, and perfusion medium.

By using the composition of the present invention, growth of animal cells may be improved, i.e., an effect of improving growth of animal cells (growth-improving effect) may be obtained. For example, by culturing animal cells with the composition of the present invention, which is a culture medium, the growth-improving effect may be obtained. Further, the growth-improving effect may be obtained by, for example, culturing animal cells in a medium to which the composition of the present invention, which is a medium additive, has been added. In other words, the composition of the present invention may have a function of improving growth of animal cells (growth-improving function). Therefore, the composition of the present invention may be used to improve growth of animal cells. That is, the composition of the present invention may be, for example, a composition for improving growth of animal cells.

By using the composition of the present invention, differentiation of animal cells may be promoted, i.e., an effect of promoting differentiation of animal cells (differentiation-promoting effect) may be obtained. For example, by culturing animal cells with the composition of the present invention, which is a culture medium, the differentiation-promoting effect may be obtained. Further, for example, by culturing animal cells in a medium to which the composition of the present invention, which is a medium additive, has been added, the differentiation-promoting effect may be obtained. In other words, the composition of the present invention may have a function of promoting differentiation of animal cells (differentiation-promoting function). Therefore, the composition of the present invention may be used to promote differentiation of animal cells. That is, the composition of the present invention may be, for example, a composition for promoting differentiation of animal cells.

By using the composition of the present invention, the growth-improving effect and differentiation-promoting effect may be obtained. That is, the composition of the present invention may be, for example, a composition for improving growth and promoting differentiation of animal cells.

In one embodiment, a product (e.g., cultured meat or target substance) may be produced by culturing animal cells. When a product is produced by culturing animal cells, the production of the product may be improved by the use of the composition of the present invention. For example, production of a product may be improved by improvement of growth of animal cells and/or promotion of differentiation of animal cells attained by the use of the composition of the present invention. That is, one embodiment of the composition of the present invention (specifically, a composition for culturing animal cell) may be a composition for improving production of a product.

The composition of the present invention may consist of the active ingredient or may contain an ingredient other than the active ingredient. The ingredient other than the active ingredient is also referred to as "additional ingredient". That is, the active ingredient may be used as the composition of the present invention as it is or as a combination with the additional ingredient. As the additional ingredient, one type of ingredient may be used, or two or more types of ingredients may be used in combination.

The additional ingredient is not particularly limited as long as the purpose of the present invention can be achieved (e.g., the growth-improving or differentiation-promoting effect can be obtained). The additional ingredient can be appropriately selected according to various conditions, for example, the type of animal cells and the manner in which the composition of the present invention is used. Examples of the additional ingredient include culture medium ingredients. Such culture medium ingredients will be described later. For example, at least when the composition of the present invention is a culture medium, the composition of the present invention may contain a culture medium ingredient. Specific examples of the additional ingredient include lecithin. That is, the composition of the present invention may contain, for example, lecithin.

The composition of the present invention may be produced in the form of, for example, formulation as appropriate. When a formulation is prepared, an additive may be used as appropriate. That is, examples of the additional ingredients also include additives used in formulations. Examples of the additives include excipients, binders, disintegrants, lubricants, stabilizers, odorants, diluents, and surfactants. The additives can be selected according to various conditions such as the form of the composition of the present invention.

The form of the composition of the present invention is not particularly limited. The composition of the present invention may be in any form, for example, powder, flake, tablet, paste, liquid, etc.

The contents and content ratios of the ingredients (i.e., active ingredient and optionally additional ingredient) in the composition of the present invention are not particularly limited as long as the purpose of the present invention can be achieved (e.g., the growth-improving or differentiation-promoting effect can be obtained). The contents and content ratios of the ingredients in the composition of the present invention can be appropriately set according to various conditions, such as the type of animal cells and the manner in which the composition of the present invention is used.

The content of the active ingredient in the composition of the present invention is more than 0% (w/w) and not more than 100% (w/w).

When the composition of the present invention contains a pea fermentation product, the content of the pea fermentation product in the composition of the present invention may be, for example, 0.1 mg/g or higher, 0.2 mg/g or higher, 0.5 mg/g or higher, 1 mg/g or higher, 2 mg/g or higher, 5 mg/g or higher, 10 mg/g or higher, 20 mg/g or higher, 50 mg/g or higher, 100 mg/g or higher, 200 mg/g or higher, 500 mg/g or higher, 1000 mg/g or higher, 2000 mg/g or higher, 5000 mg/g or higher, 10000 mg/g or higher, 20000 mg/g or higher, or 50000 mg/g or higher, and may be 100000 mg/g or lower, 50000 mg/g or lower, 20000 mg/g or lower, 10000 mg/g or lower, 5000 mg/g or lower, 2000 mg/g or lower, 1000 mg/g or lower, 500 mg/g or lower, 200 mg/g or lower, 100 mg/g or lower, 50 mg/g or lower, 20 mg/g or lower, 10 mg/g or lower, 5 mg/g or g or lower, 2 mg/g or lower, 1 mg/g or lower, 0.5 mg/g or lower, or 0.2 mg/g or lower, or may be any non-contradictory combination of these minimum and maximum contents. The content of the pea fermentation product in the composition of the present invention may specifically be, for example, 0.1 to 0.2 mg/g, 0.2 to 0.5 mg/g, 0.5 to 1 mg/g, 1 to 2 mg/g, 2 to 5 mg/g, 5 to 10 mg/g, 10 to 20 mg/g, 20 to 50 mg/g, 50 to 100 mg/g, 100 to 200 mg/g, 200 to 500 mg/g, 500 to 1000 mg/g, 1000 to 2000 mg/g, 2000 to 5000 mg/g, 5000 to 10000 mg/g, 10000 to 20000 mg/g, 20000 to 50000 mg/g, or 50000 to 100000 mg/g. The content of the pea fermentation product in the composition of the present invention may specifically be, for example, 0.1 to 100000 mg/g, 0.2 to 20000 mg/g, 0.5 to 5000 mg/g, or 1 to 1000 mg/g. The "content of the pea fermentation product" shall mean the content of the pea fermentation product in terms of the content in the original culture, the content of the pea fermentation product in terms of the content in the original culture supernatant, or the content of the pea fermentation product in terms of the content in a product provided and distributed as a commercial product, unless especially noted. That is, for example, if it is expressed that "the content of the pea fermentation product is 0.1 mg/g or higher", it is sufficient that at least one of the content of the pea fermentation product in terms of the content thereof in the original culture, the content of the pea fermentation product in terms of the content in the original culture supernatant, or the content of the pea fermentation product in terms of the content in a product provided and distributed as a commercial product is 0.1 mg/g or higher, unless especially noted. The "original culture" means culture in which concentrations of the ingredients are not changed by concentration, dilution, or the like after culturing, and it may specifically mean culture at the end of the culture. The original culture is also referred to as "original fermentation product". The "original culture supernatant" means supernatant of the culture in which concentrations of the ingredients are not changed by concentration, dilution, or the like after culturing, except for solid-liquid separation, and may specifically mean supernatant obtained by solid-liquid separation of the culture at the end of the culture. Examples of the pea fermentation product provided and distributed as a commercial product include commercially available pea-based soy sauce (soy-free). A content higher than 100% (w/w) (i.e., higher than 1000 mg/g) means that the culture is contained in the composition of the present invention in a concentrated state. That is, for example, a content of 2000 mg/g means that the culture is concentrated 2-fold after culturing and is contained in the composition of the present invention.

When the composition of the present invention contains a pea protein, the content of the pea protein in the composition of the present invention may be, for example, 0.05 mg/g or higher, 0.1 mg/g or higher, 0.2 mg/g or higher, 0.5 mg/g or higher, 1 mg/g or higher, 2 mg/g or higher, 5 mg/g or higher, 10 mg/g or higher, 20 mg/g or higher, 50 mg/g or higher, 100 mg/g or higher, 200 mg/g or higher, 500 mg/g or higher, 700 mg/g or higher, or 900 mg/g or higher, and may be 1000 mg/g or lower, 900 mg/g or lower, 700 mg/g or lower, 500 mg/g or lower, 200 mg/g or lower, 100 mg/g or lower, 50 mg/g or lower, 20 mg/g or lower, 10 mg/g or lower, 5 mg/g or lower, 2 mg/g or lower, 1 mg/g or lower, 0.5 mg/g or lower, 0.2 mg/g or lower, or 0.1 mg/g or lower, or may be any non-contradictory combination of these minimum and maximum contents. The content of pea protein in the composition of the present invention may specifically be, for example, 0.05 to 0.1 mg/g, 0.1 to 0.2 mg/g, 0.2 to 0.5 mg/g, 0.5 to 1 mg/g, 1 to 2 mg/g, 2 to 5 mg/g, 5 to 10 mg/g, 10 to 20 mg/g, 20 to 50 mg/g, 50 to 100 mg/g, 100 to 200 mg/g, 200 to 500 mg/g, 500 to 700 mg/g, 700 to 900 mg/g, or 900 to 1000 mg/g. The content of pea proteins in the composition of the present invention may specifically be, for example, 0.05 to 1000 mg/g, 0.1 to 1000 mg/g, 0.2 to 1000 mg/g, or 0.5 to 1000 mg/g.

When the composition of the present invention contains yeast extract, the content of the yeast extract in the composition of the present invention may be, for example, 0.01 mg/g or higher, 0.02 mg/g or higher, 0.05 mg/g or higher, 0.1 mg/g or higher, 0.2 mg/g or higher, 0.5 mg/g or higher, 1 mg/g or higher, 2 mg/g or higher, 5 mg/g or higher, 10 mg/g or higher, 20 mg/g or higher, 50 mg/g or higher, 100 mg/g or higher, 200 mg/g or higher, 500 mg/g or higher, 700 mg/g or higher, or 900 mg/g or higher, and may be 1000 mg/g or lower, 900 mg/g or lower, 700 mg/g or lower, 500 mg/g or lower, 200 mg/g or lower, 100 mg/g or lower, 50 mg/g or lower, 20 mg/g or lower, 10 mg/g or lower, 5 mg/g or lower, 2 mg/g or lower, 1 mg/g or lower, 0.5 mg/g or lower, 0.2 mg/g or lower, 0.1 mg/g or lower, 0.05 mg/g or lower, or 0.02 mg/g or lower, or may be any non-contradictory combination of these minimum and maximum contents. The content of the yeast extract in the composition of the present invention may specifically be, for example, 0.01 to 0.02 mg/g, 0.02 to 0.05 mg/g, 0.05 to 0.1 mg/g, 0.1 to 0.2 mg/g, 0.2 to 0.5 mg/g, 0.5 to 1 mg/g, 1 to 2 mg/g, 2 to 5 mg/g, 5 to 10 mg/ g, 10 to 20 mg/g, 20 to 50 mg/g, 50 to 100 mg/g, 100 to 200 mg/g, 200 to 500 mg/g, 500 to 700 mg/g, 700 to 900 mg/g, or 900 to 1000 mg/g. The content of the yeast extract in the composition of the present invention may specifically be, for example, 0.01 to 1000 mg/mL, 0.02 to 1000 mg/mL, 0.05 to 1000 mg/mL, or 0.1 to 1000 mg/mL. The term "content of yeast extract" means the content of yeast extract calculated on the basis of dry weight of the yeast extract.

The content of the additional ingredient in the composition of the present invention is lower than 100% (w/w).

When the composition of the present invention contains lecithin, the content of the lecithin in the composition of the present invention may be, for example, 0.1 µg/g or higher, 0.2 µg/g or higher, 0.5 µg/g or higher, 1 µg/g or higher, 2 µg/g or higher, 5 µg/g or higher, 10 µg/g or higher, 20 µg/g or higher, 50 µg/g or higher, 100 µg/g or higher, 1 mg/g or higher, 10 mg/g or higher, or 100 mg/g or higher, and may be lower than 1000 mg/g, 100 mg/g or lower, 10 mg/g or lower, 1 mg/g or lower, 100 µg/g or lower, 50 µg/g or lower, 20 µg/g or lower, 10 µg/g or lower, 5 µg/g or lower, 2 µg/ g or lower, 1 µg/g or lower, 0.5 µg/g or lower, or 0.2 µg/g or lower, or may be any non-contradictory combination of these minimum and maximum contents. The content of the active ingredient in the composition of the present invention may be, for example, 0.1 to 0.2 µg/g, 0.2 to 0.5 µg/g, 0.5 to 1 µg/g, 1 to 2 µg/g, 2 to 5 µg/g, 5 to 10 µg/g, 10 to 20 µg/g, 20 to 50 µg/g, 50 to 100 µg/g, 100 µg/g to 1 mg/g, 1 to 10 mg/g, 10 to 100 mg/g, or 100 mg/g or higher and lower than 1000 mg/g. The content of the active ingredient in the composition of the present invention may specifically be, for example, 0.1 µg/g or higher and lower than 1000 mg/g, 0.2 µg/g or higher and lower than 1000 mg/g, 0.5 µg/g or higher and lower than 1000 mg/g, or 1 µg/g or higher and lower than 1000 mg/g.

In one embodiment, the composition of the present invention may be substantially free of serum. The expression that "the composition of the present invention is substantially free of serum" may mean that the content of serum in the composition of the present invention is 1% (w/w) or lower, 0.1% (w/w) or lower, 0.01% (w/w) or lower, or 0.001% (w/w) or lower, and may also include cases where the content of serum in the composition of the present invention is 0 (zero) (i.e., the composition of the present invention does not contain serum).

In one embodiment, the composition of the present invention may be substantially free of animal-derived albumin. The expression that "the composition of the present invention is substantially free of animal-derived albumin" may mean that the content of animal-derived albumin in the composition of the present invention is 1% (w/w) or lower, 0.1% (w/w) or lower, 0.01% (w/w) or lower, or 0.001% (w/w) or lower, and may include cases in which the content of animal-derived albumin in the composition of the present invention is 0 (zero) (i.e., the composition of the present invention does not contain animal-derived albumin).

The contents of the respective ingredients (i.e., the active ingredient and optionally additional ingredient) in the composition of the present invention can be set so that, for example, the concentrations of the respective ingredients in the medium in the method of the present invention described later, can be achieved.

If the composition of the present invention contains two or more types of ingredients, they may be contained in the composition of the present invention as a mixture, separately, or separately in any combinations thereof. For example, the composition of the present invention may be provided as a set of a package of the active ingredient and a package of the additional ingredient. In such a case, the ingredients in the set may be used together as appropriate at the time of use.

### <3> Method of the present invention

The method of the present invention is a method for culturing animal cells, comprising the step of culturing the animal cells in the presence of the active ingredient (i.e., pea-derived material and/or yeast extract). This step is also referred to as the "animal cell-culturing step".

By utilizing the active ingredient, specifically by culturing animal cells in the presence of the active ingredient, growth of animal cells may be improved, i.e., an effect of improving growth of animal cells (growth-improving effect) may be obtained. That is, one embodiment of the method of the present invention (specifically, a method for culturing animal cells) may be, for example, a method for improving growth of animal cells.

By utilizing the active ingredient, specifically by culturing animal cells in the presence of the active ingredient, differentiation of animal cells may be promoted, i.e., an effect of promoting differentiation of animal cells (differentiation-promoting effect) may be obtained. That is, an embodiment of the method of the present invention (specifically, a method for culturing animal cells) may be, for example, a method for promoting differentiation of animal cells.

By utilizing the active ingredient, specifically by culturing animal cells in the presence of the active ingredient, the growth-improving effect and the differentiation-promoting effect may be obtained. That is, an embodiment of the method of the present invention (specifically, a method for culturing animal cells) may be, for example, a method for improving growth and promoting differentiation of animal cells.

The purpose of culturing animal cell is not particularly limited.

In one embodiment, a product (e.g., cultured meat or target substance) may be produced by culturing animal cells. That is, if the animal cells can form cultured meat by culturing, cultured meat can be produced by culturing the cells. Cultured meat may consist primarily of muscle tissues. Therefore, the expression that "animal cells can form cultured meat by culturing " may mean that, for example, animal cells can differentiate and form muscle tissues when they are cultured, or specifically, animal cells can differentiate into myotube cells and form muscle tissues when they are cultured. If the animal cells have an ability to produce a target substance, the target substance can be produced by culturing the cells. That is, one embodiment of the method for the present invention (specifically, a method for culturing animal cells) may be a method for producing a product, comprising the step of culturing animal cells in the presence of the active ingredient. The step of culturing animal cells may specifically be a step of producing a product by culturing animal cells in the presence of the active ingredient. In the method for producing cultured meat, the cultured animal cells may form cultured meat. In the method for producing a target substance, the cultured animal cells may produce the target substance. In the case of producing a product by culturing animal cells, the production of the product may be improved by the use of the active ingredient. For example, the production of a product may be improved by improving growth and/or promoting differentiation of the animal cells through the use of the active ingredient. That is, an embodiment of the method for the present invention (specifically, a method for culturing animal cells) may be a method for improving production of a product.

The term "animal cells" means cells of animal. The animal cells are not particularly limited. The animal cells can be selected according to various conditions, such as, for example, the purpose of culturing animal cells. For example, when cultured meat is produced by culturing animal cells, the animal cells are not particularly limited as long as cultured meat can be formed by culturing them. The term "animal" may mean any organism classified in the kingdom *Animalia.* Examples of the animal include vertebrates and aquatic organisms. Examples of the vertebrates include mammals, birds, reptiles, amphibians, and fish. As the vertebrates, mammals, birds, and fish can be especially mentioned. As the vertebrates, mammals can be especially mentioned. Examples of the mammals include primates such as humans, monkeys, and chimpanzees; rodents such as hamsters, mice, rats, and guinea pigs; other land mammals such as cattle, pigs, sheep, goats, rabbits, horses, deer, water buffalos, reindeer, donkeys, camels, dogs, and cats; and aquatic mammals such as whales, dolphins, reindeer, and sea lions. Examples of the birds include chickens, turkeys, ducks, geese, guinea fowls, quails, and ostriches. Examples of the fish include eels, tuna, longtooth grouper, sea bream, salmon, cod, and puffer fish. In addition to aquatic mammals and fish, aquatic organisms include crustaceans such as shrimp and crabs, shellfish such as scallops and oysters, and other aquatic organisms such as squid and octopus. Fish and crustaceans are especially mentioned as the aquatic organisms. Examples of animals suitable for the production of cultured meat include animals for meat. Examples of the animals for meat include any of the above-mentioned animals whose meat is edible. As the animals for meat (especially mammals), cattle, pigs, sheep, goats, and rabbits can be especially mentioned. As the animals (especially mammals) for meat, cattle and pigs can be more especially mentioned. As the animals (especially mammals) for meat, cattle can be further especially mentioned. As the animals for meat (especially birds), chickens, turkeys, ducks, geese, guinea fowls, quails, and ostriches can be particularly mentioned. As the animals for meat (especially birds), chickens can be more particularly mentioned. As the animals for meat (especially aquatic organisms), fish and crustaceans exemplified above can be especially mentioned. As the animals for meat (especially aquatic organisms), eels, tunas, and shrimps can be more especially mentioned. The tissues or cells from which the animal cells are derived are not particularly limited. Examples of the tissues or cells from which the animal cells are derived include ovary, kidney, adrenal gland, tongue epithelium, olfactory epithelium, pineal gland, thyroid gland, melanocytes, skin, spleen, liver, lung, pancreas, uterus, stomach, colon, small intestine, large intestine, bladder, prostate, testis, thymus gland, muscle, connective tissue, bone, cartilage, vascular tissue, blood (including cord blood), bone marrow, heart, eye, brain, and nerve tissue. The animal cells may or may not have differentiated. Specific examples of the animal cells include germ cells, somatic cells, stem cells, and progenitor cells. Examples of the germ cells include sperm and oocytes. Examples of the somatic cells include myoblasts, fibroblasts, bone marrow cells, B lymphocytes, T lymphocytes, neutrophils, red blood cells, platelets, macrophages, monocytes, osteocytes, pericytes, dendritic cells, adipocytes, mesenchymal cells, epithelial cells, epidermal cells (e.g., keratinocytes, corneocytes etc.), endothelial cells, vascular endothelial cells, liver parenchymal cells, chondrocytes, cumulus cells, nerve cells, glial cells, oligodendrocytes, microglia, astrocytes, cardiac cells, esophageal cells, muscle cells (e.g., smooth muscle cells and skeletal muscle cells), pancreatic beta cells, melanocytes, and mononuclear cells. Examples of the stem cells include adult stem cells such as hematopoietic stem cells, satellite cells (muscle stem cells), neural stem cells, mesenchymal stem cells, mammary stem cells, olfactory mucosa stem cells, neural crest stem cells, liver stem cells, pancreatic stem cells, germline stem cells, intestinal stem cells, and hair follicle stem cells; pluripotent stem cells such as embryonic stem cells (ES cells), embryonic tumor cells, embryonic germ cells, and induced pluripotent stem cells (iPS cells); and cancer stem cells. Examples of the progenitor cells include satellite cells, pancreatic progenitor cells, vascular progenitor cells, vascular endothelial progenitor cells, and hematopoietic progenitor cells (such as CD34-positive cells derived from umbilical cord blood). Preferred examples of the animal cells suitable for the production of cultured meat include stem cells such as satellite cells (muscle stem cells), mesenchymal stem cells, embryonic stem cells (ES cells), and induced pluripotent stem cells (iPS cells), as well as myoblasts. Especially preferred examples of the animal cells suitable for the production of cultured meat include satellite cells (muscle stem cells) and myoblasts. Myoblasts may be classified as stem cells.

Examples of the animal cells also include MyoD+ cells (i.e., cells expressing MyoD) and PAX7+ cells (i.e., cells expressing PAX7). The MyoD+ cells may be, for example, myoblasts. The PAX7+ cells may be, for example, satellite cells. The satellite cells may be activated. Examples of the MyoD+ cells and PAX7+ cells include MyoD+ cells and PAX7+ cells of the animal species exemplified above. As the PAX7+ cells, PAX7+ cells of birds such as chickens can be especially mentioned. The animal cells may be MyoD+ cells, for example, when a growth-improving effect is to be obtained. The animal cells may be, for example, PAX7+ cells when a differentiation-promoting effect is to be obtained. The animal cells may specifically be, for example, PAX7+ cells of birds such as chickens when a differentiation-promoting effect is to be obtained.

For "the animal cells satisfy a certain condition", it is sufficient that the animal cells satisfy the condition during at least a part of the culture period, and it is not necessary that the animal cells satisfy the condition during the entire culture period. That is, the expression that "the animal cells satisfy a certain condition" means not only the case where the animal cells satisfy the condition at the start of the culture (i.e., the culture is started with animal cells that satisfy the condition), but may also mean that the animal cells satisfy the condition after the start of the culture. For example, the expression that "the animal cells are MyoD+ cells" means not only that the culture is started with MyoD+ animal cells, but may also mean that the animal cells become MyoD+ after the culture is started.

The culture of animal cells is performed in the presence of the active ingredient. The expression that "the culture of animal cells is performed in the presence of the active ingredient" may mean that, for example, the animal cells are cultured in a medium containing the active ingredient. The expression that "the culture of the animal cells is performed in the presence of the active ingredient" may mean that, for example, the active ingredient is supplied to the culture system during culturing the animal cells.

The active ingredient may be used for culturing animal cells, for example, in the form of the composition of the present invention. The composition of the present invention may be used for culturing animal cells as, for example, a culture medium. That is, for example, when the composition of the present invention is a culture medium, animal cells may be cultured in the composition of the present invention (i.e., culture medium). That is, the expression that "animal cells are cultured in a medium containing the active ingredient" may also mean that the animal cells are cultured in the composition of the present invention that is a culture medium. The composition of the present invention may also be used for culturing animal cells, for example, as a medium additive. That is, for example, when the composition of the present invention is a medium additive, the composition of the present invention (i.e., medium additive) may be added to the medium, and the animal cells may be cultured in the medium to which the composition of the present invention has been added. That is, the expression that "animal cells are cultured in a medium containing the active ingredient" may also mean that the animal cells are cultured in a medium to which the composition of the present invention that is a medium additive has been added. Furthter, for example, the composition of the present invention or a medium to which it has been added may be supplied to the culture system during culturing animal cells. That is, the expression that "the active ingredient is supplied to the culture system during culturing animal cells" may also mean that the composition of the present invention or a medium to which it has been added is supplied to the culture system during culturing animal cells.

The medium composition and culture conditions are not particularly limited, except that the culturing of animal cells is performed in the presence of the active ingredient as long as the purpose of culturing animal cells can be achieved. The medium composition and culture conditions can be appropriately set according to various conditions, such as the type of animal cells and the purpose of culturing animal cells. For example, when cultured meat is produced by culturing animal cells, the medium composition and culture conditions are not particularly limited as long as cultured meat can be formed by culturing animal cells. For example, the animal cells can be cultured by using a conventional culture medium and conventional conditions used for culturing animal cells as they are or with any appropriate modification, except that the culture is performed in the presence of the active ingredient. When cultured meat is produced by culturing animal cells, the animal cells can be cultured, for example, by using a conventional medium and conventional conditions used for the formation of muscle tissues by differentiation of animal cells (e.g., production of cultured meat) as they are or with any appropriate modification, except that the culture is performed in the presence of the active ingredient.

The culture can be performed, for example, by using a liquid medium. The culture can be performed as batch culture, fed-batch culture, continuous culture, or a combination thereof. Examples of the continuous culture include perfusion culture and chemostat culture. The culture medium at the start of culture is also called "starting medium" or "basal medium". The medium supplied to the culture system (e.g., starting medium) in fed-batch culture is also referred to as "feed medium". The medium supplied to the culture system (e.g., starting medium) in continuous culture (which is not limited to perfusion culture) is also referred to as "perfusion medium". Supplying feed medium or perfusion medium to the culture system in the fed-batch culture or continuous culture is also referred to simply as "medium supply". The medium may be supplied throughout the entire culture period or during only a part of the culture period. The medium may be supplied continuously or intermittently. During the culture (especially continuous culture such as perfusion culture), the culture medium may be withdrawn. The medium may be withdrawn throughout the entire culture period or during only a part of the culture period. The medium may be withdrawn continuously or intermittently. The medium may or may not be simultaneously withdrawn and supplied. The culture vessel may be coated with cell adhesion molecules, e.g., fibronectin. The culture may be performed three-dimensionally, e.g., by using a scaffold material. When cultured meat is produced by culturing animal cells, the culture may be performed three-dimensionally, for example, by using a scaffold material that matches the shape of the cultured meat to be formed.

The medium used for the culture can be selected independently, for example, for the basal medium, feed medium, and perfusion medium.

The medium used for the culture may be a commercially available medium or a medium appropriately prepared.

The medium used for the culture is, for example, a medium containing essential ingredients for culturing animal cells (e.g., carbon source, nitrogen source, inorganic salts, etc.).

Specific examples of the medium used for the culture include Dulbecco's Modified Eagle's Medium (DMEM), Ham's Nutrient Mixture F12, DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB131 medium, William's Medium E, and Fischer's Medium.

Specific examples of the medium used for the culture (which may be especially, for example, a medium used for culturing stem cells (especially pluripotent stem cells)) also include STEMPRO (registered trademark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trademark) Serum-Free Medium for hES cells (Millipore), PluriSTEM (trademark) Human ES/iPS Medium (EMD Millipore), NutriStem (trademark) hESC XF medium (Biological Industries Israel Beit-Haemek), NutriStem (trademark) XF/FF Culture Medium (Stemgent), AF NutriStem (registered trademark) hESC XF Medium (Biological Industries Israel Beit-Haemek), S-medium (DS Pharma Biomedical Corporation), StemFit (registered trademark) AK03N medium (Ajinomoto Co., Ltd.), hESF9 medium, hESF-FX medium, CDM medium, DEF-CS 500 Xeno-Free 3D Spheroid Culture Medium (Cellartis), StemFlex medium (Thermo Fisher Scientific), etc.

Other examples of commercially available media for culturing animal cell include CELLiST Basal Media BASAL3, BASAL4P, and BASAL10 (Ajinomoto Co., Ltd.), Opti-MEM (Thermo Fisher Scientific), RPMI 1640 (Thermo Fisher Scientific), CD293 (Thermo Fisher Scientific), CHO-S-SFMII (Thermo Fisher Scientific), CHO-SF (Sigma-Aldrich), EX-CELL CD CHO (Sigma-Aldrich), EX-CELLTM302 (Sigma-Aldrich), IS CHO-CD (Irvine Scientific), IS CHO-CDXP (Irvine Scientific), etc.

Such culture media as exemplified above may be used for the culture, for example, by adding the active ingredient to them.

The culture medium used for the culture may be, for example, the composition of the present invention (specifically, the composition of the present invention that is a culture medium). That is, when the composition of the present invention is a culture medium, the composition of the present invention may be used for the culture as it is or after it is prepared as a liquid medium having a desired ingredient composition as appropriate. For example, the composition of the present invention may be prepared as a liquid medium by dilution with an aqueous medium such as water or aqueous buffer solution, and used for the culture.

The medium used for the culture may be, for example, a medium to which the composition of the present invention (specifically, the composition of the present invention that is a medium additive) has been added. The medium to which the composition of the present invention is to be added may be a commercially available medium or a medium prepared as appropriate.

The culture medium may contain various culture medium ingredients. Examples of the medium ingredients include carbon sources, amino acid sources, peptides, proteins, vitamins, fatty acids, lipids, inorganic ingredients, pH buffers, growth factors, cytokines, hormones, cell adhesion factors, extracellular matrix components, serum, lecithin, antibiotics, and gene expression inducers. Any of these medium ingredients can be essential or effective for, for example, survival or proliferation of animal cells. Any of these medium ingredients may be contained in, for example, such media as exemplified above beforehand, or may be added to such media as exemplified above.

Examples of the carbon sources include sugars such as glucose, fructose, sucrose, and maltose.

Examples of the amino acids sources include amino acids. Both peptides and proteins can also be examples of the amino acid sources. Examples of the amino acids include glycine, alanine, valine, leucine, isoleucine, cysteine, methionine, phenylalanine, tyrosine, tryptophan, histidine, lysine, arginine, serine, threonine, aspartic acid, glutamic acid, asparagine, glutamine, proline, and ornithine. The amino acids may be, for example, L-amino acids.

Examples of the peptides include dipeptides and tripeptides. Specific examples of the peptides include glycyl-glycyl-glycine and soy peptides. The descriptions for amino acids can also be applied to amino acids constituting peptides.

Examples of the proteins include albumin and transferrin.

Examples of the vitamins include vitamin A, vitamin B1, vitamin B2, vitamin B3, vitamin B5, vitamin B6, vitamin B7, vitamin B9, vitamin B12, vitamin C, vitamin D, vitamin E, vitamin K and precursors thereof.

Examples of the fatty acids include oleic acid, arachidonic acid, and linoleic acid.

Examples of the lipids include cholesterol.

Examples of the inorganic ingredients include sodium, potassium, calcium, magnesium, phosphorus, and various trace elements (e.g., Co, Cu, F, Fe, Mn, Mo, Ni, Se, Si, Ni, Bi, V, and Zn). Specific examples of the inorganic ingredients include inorganic salts such as sodium chloride, potassium chloride, calcium chloride, magnesium sulfate, and sodium dihydrogenphosphate.

Examples of the pH buffers include sodium hydrogencarbonate, phosphoric acid salts, N, N-bis(2-hydroxyethyl)- 2-aminoethanesulfonic acid (BES), 4-(2-hydroxyethyl)-1-piperazineethanesulfonic acid (HEPES), and N-[tris(hydroxymethyl)methyl]glycine (tricine).

Examples of the growth factors include fibroblast growth factor (FGF), hepatocyte growth factor (HGF), epidermal growth factor (EGF), transforming growth factor-α (TGF-α)), transforming growth factor-β (TGF-β), vascular endothelial growth factor (VEGF), activin A, and insulin-like growth factor-1 (IGF-1). Examples of FGF include basic fibroblast growth factor (bFGF). As the growth factor, bFGF can be especially mentioned. That is, as the medium ingredient, at least bFGF may be used. That is, the medium may contain at least bFGF. Examples of bFGF include bovine basic fibroblast growth factor (bbFGF). The term "bovine basic fibroblast growth factor (bbFGF)" may refer to bovine-derived bFGF. The term "bovine" used for bbFGF may refer to an organism of the genus *Bos.* Examples of the organism of the genus *Bos* include *Bos taurus.* bbFGF is not limited to bFGF found in organisms of the genus *Bos,* but may also be a modified version thereof.

Examples of the cytokines include interleukins.

Examples of the hormones include dexamethasone, hydrocortisone, estradiol, progesterone, glucagon, and insulin.

Examples of the cell adhesion factors or extracellular matrix components include type I collagen, type II collagen, fibronectin, laminin, poly-L-lysine, and poly-D-lysine.

Examples of the antibiotics include amphotericin B, kanamycin, gentamicin, streptomycin, and penicillin.

In one embodiment, the medium may be substantially free of serum. For example, the medium may be substantially free of serum during the entire culture period. The expression that "the medium substantially free of serum" may mean that the serum content in the medium is 1% (w/w) or lower, 0.1% (w/w) or lower, 0.01% (w/w) or lower, or 0.001% (w/w) or lower, and may also mean that the serum content in the medium is 0 (zero) (i.e., the medium does not contain serum).

In one embodiment, the medium may be substantially free of animal-derived albumin. Examples of the animal from which the albumin is derived include the animals exemplified for animal cells. For example, the medium may be substantially free of animal-derived albumin during the entire culture period. The expression that "the medium substantially free of animal-derived albumin" may mean that the content of animal-derived albumin in the medium is 1% (w/w) or lower, 0.1% (w/w) or lower, 0.01% (w/w) or lower, or 0.001% (w/w) or lower, and may also mean that the content of animal-derived albumin in the medium is 0 (zero) (i.e., the medium does not contain animal-derived albumin).

Any of the various ingredients, such as the active ingredient, may be contained in the starting medium, feed medium, perfusion medium, or a combination thereof. That is, the various ingredients such as the active ingredient may be supplied to the medium individually or in any combination during the culture process. Any of these ingredients may be supplied once or multiple times or continuously. The ingredient compositions (e.g., types and/or concentrations of ingredients contained) of the starting medium, feed medium, and perfusion medium may or may not be the same. That is, the types of ingredients contained in the starting medium may or may not be the same as the types of ingredients contained in the feed medium or perfusion medium. The concentrations of ingredients contained in the starting medium may or may not be the same as the concentrations of the ingredients contained in the feed medium or perfusion medium. For example, if a feed medium is used for perfusion culture, the ingredient compositions of the starting medium and the feed medium may be the same. Two or more types of feed media or perfusion media having different ingredient compositions (e.g., types and/or concentrations of ingredients contained) may also be used. For example, if the feed medium or perfusion medium is intermittently supplied multiple times, the ingredient compositions of the feed medium or perfusion medium for respective times of the supply may or may not be the same. In addition, any of the various ingredients such as the active ingredient may be supplied to the medium in such a form that is not contained in the feed medium or perfusion medium, such as in the form of powder.

Inoculation amount of the animal cells at the start of the culture may be, for example, 1 x 10² cells/mL or more, 1 x 10³ cells/mL or more, 1 x 10⁴ cells/mL or more, 1 x 10⁵ cells/mL or more, 1 x 10⁶ cells/mL or more, or 1 x 10⁷ cells/mL or more, and may be 1 x 10⁸ cells/mL or less, 1 x 10⁷ cells/mL or less, 1 x 10⁶ cells/mL or less, 1 x 10⁵ cells/mL or less, 1 x 10⁴ cells/mL or less, or 1 x 10³ cells/mL or less, in terms of viable cell count, or may be any non-contradictory combination of there minimum and maximum amounts. Specifically, the inoculation amount of the animal cells at the start of the culture may be, for example, 1 x 10² to 1 x 10³ cells/mL, 1 x 10³ to 1 x 10⁴ cells/mL, 1 x 10⁴ to 1 x 10⁵ cells/mL, 1 x 10⁵ to 1 x 10⁶ cells/mL, 1 x 10⁶ to 1 x 10⁷ cells/mL, or 1 x 10⁷ to 1 x 10⁸ cells/mL, in terms of viable cell count. The inoculation amount of the animal cells at the start of the culture may specifically be, for example, 1 x 10² to 1 x 10⁸ cells/mL, 1 x 10³ to 1 x 10⁷ cells/mL, or 1 x 10³ to 1 x 10⁶ cells/mL in terms of viable cell count. The viable cell count can be measured by using, for example, the live/dead cell autoanalyzer, Vi-CELL^{™} XR (Beckman Coulter).

The culture may be performed, for example, in a CO₂-containing atmosphere such as 5 to 15% CO₂. The pH of the medium may be, for example, around neutral. The term "around neutral" means, for example, pH 6 to 8, pH 6.5 to 7.5, or pH 6.8 to 7.2. During the culture, the pH of the medium may be adjusted as needed. The pH of the medium can be adjusted by using various alkaline or acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 30 to 38°C. The culture period may be, for example, 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, 15 days or longer, or 20 days or longer, and may be 60 days or shorter, 50 days or shorter, 40 days or shorter, 30 days or shorter, 25 days or shorter, 20 days or shorter, 15 days or shorter, 12 days or shorter, 10 days or shorter, 9 days or shorter, 8 days or shorter, or 7 days or shorter, or may be any non-contradictory combination of these minimum and maximum periods. The culture period may specifically be, for example, 1 to 60 days, 3 to 25 days, or 5 to 20 days. The culture may be continued, for example, until a product is produced to a desired degree.

If the medium contains a pea fermentation product, the concentration of the pea fermentation product in the medium may be, for example, 0.1 mg/mL or higher, 0.2 mg/mL or higher, 0.5 mg/mL or higher, 1 mg/mL or higher, 2 mg/mL or higher, 5 mg/mL or higher, 10 mg/mL or higher, 20 mg/mL or higher, or 50 mg/ mL or higher, and may be 100 mg/mL or lower, 50 mg/mL or lower, 20 mg/mL or lower, 10 mg/mL or lower, 5 mg/mL or lower, 2 mg/mL or lower, 1 mg/mL or lower, 0.5 mg/mL or lower, or 0.2 mg/mL or lower, or any non-contradictory combination of these minimum and maximum concentrations. The concentration of the pea fermentation product in the medium may specifically be, for example, 0.1 to 0.2 mg/mL, 0.2 to 0.5 mg/mL, 0.5 to 1 mg/mL, 1 to 2 mg/mL, 2 to 5 mg/mL, 5 to 10 mg/mL, 10 to 20 mg/mL, 20 to 50 mg/mL, or 50 to 100 mg/mL. The concentration of the pea fermentation product in the medium may specifically be, for example, 0.1 to 100 mg/mL, 0.2 to 50 mg/mL, 0.5 to 20 mg/mL, or 1 to 10 mg/mL. Unless especially noted, the term "concentration of the pea fermentation product" shall mean the concentration of the pea fermentation product in terms of the concentration in the original culture, the concentration of the pea fermentation product in terms of the concentration in the original culture supernatant, or the concentration of the pea fermentation product in terms of the concentration in a product that is commercially provided and distributed. That is, for example, when it is expressed that "the concentration of the pea fermentation product is 0.1 mg/mL or higher", it is sufficient that at least one of the concentration of the pea fermentation product in terms of the concentration in the original culture, the concentration of the pea fermentation product in terms of the concentration in the original culture supernatant, and the concentration of the pea fermentation in terms of the concentration in a product commercially provided and distributed is 0.1 mg/mL or higher, unless especially specified. For example, if the culture is concentrated 2-fold after culturing and added to the medium at a concentration of 0.1 mg/mL, the concentration of the pea fermentation product in the medium is 0.2 mg/mL in terms of the concentration in the original culture.

When the medium contains a pea protein, the concentration of the pea protein in the medium may be, for example, 0.05 mg/mL or higher, 0.1 mg/mL or higher, 0.2 mg/mL or higher, 0.5 mg/mL or higher, 1 mg/mL or higher, 2 mg/mL or higher, 5 mg/mL or higher, 7 mg/mL or higher, 10 mg/mL or higher, 15 mg/mL or higher, 20 mg/mL or higher, or 30 mg/mL or higher, and may be 50 mg/mL or lower, 30 mg/mL or lower, 20 mg/mL or lower, 15 mg/mL or lower, 10 mg/mL or lower, 7 mg/mL or lower, 5 mg/mL or lower, 2 mg/mL or lower, 1 mg/mL or mL or lower, 0.5 mg/mL or lower, 0.2 mg/mL or lower, or 0.1 mg/mL or lower, or may be any non-contradictory combination of these minimum and maximum concentrations. The concentration of the pea protein in the medium may specifically be, for example, 0.05 to 0.05 mg/mL, 0.1 to 0.1 mg/mL, 0.2 to 0.2 mg/mL, 0.5 to 0.5 mg/mL, 1 to 1 mg/mL, 2 to 2 mg/mL, 5 to 5 mg/mL, 7 to 7 mg/mL, 10 to 10 mg/ mL, 15 to 15 mg/mL, 20 to 20 mg/mL, or 30 to 50 mg/mL. The concentration of the pea proteins in the medium may specifically be, for example, 0.05 to 50 mg/mL, 0.1 to 30 mg/mL, 0.2 to 20 mg/mL, or 0.5 to 15 mg/mL.

When the medium contains yeast extract, the concentration of the yeast extract in the medium may be, for example, 0.01 mg/mL or higher, 0.02 mg/mL or higher, 0.05 mg/mL or higher, 0.1 mg/mL or higher, 0.2 mg/mL or higher, 0.5 mg/mL or higher, 1 mg/mL or higher, 2 mg/mL or higher, or 5 mg/ mL or higher, and may be 10 mg/mL or lower, 5 mg/mL or lower, 2 mg/mL or lower, 1 mg/mL or lower, 0.5 mg/mL or lower, 0.2 mg/mL or lower, 0.1 mg/mL or lower, 0.05 mg/mL or lower, or 0.02 mg/mL or lower, or may be any non-contradictory combination of these minimum and maximum concentrations. The concentration of the yeast extract in the medium may specifically be, for example, 0.01 to 0.02 mg/mL, 0.02 to 0.05 mg/mL, 0.05 to 0.1 mg/mL, 0.1 to 0.2 mg/mL, 0.2 to 0.5 mg/mL, 0.5 to 1 mg/mL, 1 to 2 mg/mL, 2 to 5 mg/mL, or 5 to 10 mg/mL. The concentration of the yeast extract in the medium may specifically be, for example, 0.01 to 10 mg/mL, 0.02 to 5 mg/mL, 0.05 to 2 mg/mL, or 0.1 to 1 mg/mL. The term "concentration of yeast extract" means the concentration of the yeast extract calculated on the basis of the dry weight of the yeast extract.

The culture of the animal cells may be performed in the presence of lecithin. The expression that "the culture of the animal cells is performed in the presence of a certain ingredient (e.g., lecithin)" may mean that, for example, the animal cells are cultured in a medium containing the ingredient. The expression that "the culture of the animal cells is performed in the presence of a certain ingredient (e.g., lecithin)" may mean that, for example, the ingredient is supplied to the culture system during the culture of the animal cells.

When the medium contains lecithin, the concentration of lecithin in the medium may be, for example, 0.1 µg/mL or higher, 0.2 µg/mL or higher, 0.5 µg/mL or higher, 1 µg/mL or higher, 2 µg/mL or higher, 5 µg/mL or higher, 7 µg/mL or higher, 10 µg/mL or higher, 15 µg/mL or higher, 20 µg/mL or higher, 30 µg/mL or higher, or 50 µg/mL or higher, and may be 100 µg/mL or lower, 50 µg/mL or lower, 30 µg/mL or lower, 20 µg/mL or lower, 15 µg/mL or lower, 10 µg/mL or lower, 7 µg/mL or lower, 5 µg/mL or lower, 2 µg/mL or lower, 1 µg/mL or lower, 0.5 µg/mL or lower, or 0.2 µg/mL or lower, or may be any non-contradictory combination of these minimum and maximum concentrations. The concentration of lecithin in the medium may specifically be, for example, 0.1 to 0.2 µg/mL, 0.2 to 0.5 µg/mL, 0.5 to 1 µg/mL, 1 to 2 µg/mL, 2 to 5 µg/mL, 5 to 7 µg/mL, 7 to 10 µg/mL, 10 to 15 µg/mL, 15 to 20 µg/mL, 20 to 30 µg/mL mL, 30 to 50 µg/mL, or 50 to 100 µg/mL. The concentration of lecithin in the medium may specifically be, for example, 0.1 to 100 µg/mL, 0.2 to 50 µg/mL, 0.5 to 30 µg/mL, or 1 to 15 µg/mL.

Any of the various ingredients such as the active ingredient may be contained in the culture medium during the entire period of the culture, or may be contained in the culture medium during only a part of the culture period. That is, the expression that "the culture is performed in a medium containing a certain ingredient" means that it is sufficient if the ingredient is contained in the medium during at least a part of the culture period, and it is not required that the ingredient be contained in the medium during the entire culture period. Any of the various ingredients such as the active ingredient may be, for example, contained in the culture medium at the start of the culture, or may be supplied to the culture medium after the start of the culture. Any of the various ingredients such as the active ingredient may be, for example, contained in the culture medium at the start of the culture and further supplied to the culture medium after the start of the culture (e.g., after consumption of the active ingredient).

Any of the various ingredients such as the active ingredient may be, for example, contained in the culture medium at the concentration exemplified above during the entire culture period, or may be contained in the culture medium at the concentration exemplified above during only a part of the culture period. That is, the expression that "the culture is performed in a medium containing a certain ingredient at a certain concentration", "a certain ingredient is contained in the medium at a certain concentration during the culture", or "concentration of a certain ingredient in the medium during the culture is at a certain concentration" means that it is sufficient that the concentration of the ingredient in the medium is within such a concentration range during at least a part of the culture period, and it is not required that the concentration of the ingredient in the culture medium is within such a concentration range during the entire culture period. Any of the various ingredients such as the active ingredient may be contained in the culture medium at the concentration exemplified above, for example, at the start of the culture, or may be supplied to the culture medium to the concentration exemplified above after the start of the culture. Further, any of the various ingredients such as the active ingredient may be contained in the medium, for example, at the concentration exemplified above at the start of the culture, and may be further supplied to the culture medium to the concentration exemplified above after the start of the culture (e.g., after consumption of the ingredient).

The length of the "part of the culture period" is not particularly limited as long as the purpose of the present invention can be achieved (e.g., the growth-improving effect or differentiation-promoting effect can be obtained). The length of the "part of the culture period" can be appropriately set according to various conditions, such as the type of the animal cells and the length of the culture period. The "part of the culture period" may be, for example, 10% or more, 20% or more, 30% or more, 40% or more, 50% or more, 60% or more, 70% or more, 80% or more, 90% or more, 95% or more, 97% or more, or 99% or more of the total culture period. The "part of the culture period" may be, for example, 10% or more, 20% or more, 30% or more, 40% or more, 50% or more, 60% or more, 70% or more, 80% or more, 90% or more, 95% or more, 97% or more, or 99% or more of the total culture period after the animal cells have differentiated into myoblasts, myocytes, or myotube cells. The "part of the culture period" may also be a period of, for example, 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, or 15 days or longer.

The concentration of any of the various ingredients such as the active ingredient in the culture medium may be set at the concentration exemplified above, for example, as an average value throughout a specific period during the culture. That is, the expression that "the culture is performed in a medium containing a certain ingredient at a certain concentration", "a certain ingredient is contained in the medium at a certain concentration during the culture", or "concentration of a certain ingredient in the medium is a certain concentration during the culture" may mean that the average concentration of the ingredient in the medium over a specific period during the culture is within such a concentration range. Although the "average value of concentration of a certain ingredient in the medium throughout a specific period of the culture" is not particularly limited as long as the variation of the concentration of the ingredient during the specific period of the culture can be monitored, it may mean, for example, average of the concentrations of the ingredient in the medium measured every 60, 30, 20, or 10 minutes during the specific period of the culture. The "specific period of the culture" can be, for example, the entire culture period or a part of the culture period. The "part of the culture period" is as described above.

Any of the various ingredients such as the active ingredient may be supplied to the culture medium throughout the entire culture period or during only a part of the culture period. The "part of the culture period" is as described above. Any of the various ingredients such as the active ingredient may be supplied to the culture medium, for example, continuously or intermittently. Any of the various ingredients such as the active ingredient may be supplied to the culture medium, for example, daily or every few days.

The concentration of any of the various ingredients such as the active ingredient in the feed medium or perfusion medium may be, for example, within any of the ranges of the concentration of the ingredient exemplified above. The concentration of any of the various ingredients such as the active ingredient in the feed medium or perfusion medium may be, for example, 1, 1.1, 1.3, 1.5, 2, 3, 5, 7, 10, 15, or 20 times of the concentration of the ingredient in the medium exemplified above or higher, and may be 100, 70, 50, 30, 20, 15, 10, 7, 5, 3, or 2 times of the same or lower, or any non-contradictory combination of these minimum and maximum concentrations. The concentration of any of the various ingredients such as the active ingredient in the feed medium or perfusion medium may specifically be, for example, 1 to 2 times, 1.1 to 2 times, 1.3 to 2 times, 1.5 to 2 times, 2 to 3 times, 3 to 5 times, 5 to 7 times, 7 to 10 times, 10 to 15 times, 15 to 20 times, 20 to 30 times, 20 to 50 times, 20 to 70, or 20 to 100 times of the concentration of the ingredient in the medium exemplified above. The concentration of any of the various ingredients such as the active ingredient in the feed medium or perfusion medium may specifically be, for example, 1 to 100 times, 2 to 50 times, or 5 to 20 times of the concentration of the ingredient in the medium exemplified above.

The concentration of any of the various ingredients such as the active ingredient can be measured by, for example, known methods used for detection or identification of compounds. Examples of such methods include HPLC, UPLC, LC/MS, GC/MS, and NMR.

The animal cells can be cultured as described above. In one embodiment, by culturing the animal cells as described above, a product (e.g., cultured meat or target substance) may be obtained. When a target substance is produced, the target substance may be accumulated in the culture, e.g., in the culture medium, on the cell surfaces, inside the cells, or in any combination thereof. The target substance may be collected from the culture as appropriate. That is, the method for producing a target substance may further comprise the step of collecting the target substance.

### <4> Use of active ingredient

The present invention also discloses the use of the active ingredient for the uses exemplified above. That is, the present invention discloses, for example, use of the active ingredient for culturing animal cells, use of the active ingredient for improving growth of animal cells, use of the active ingredient for promoting differentiation of animal cells, and use of the active ingredient in manufacture of the composition of the present invention such as the composition for culturing animal cell.

The present invention also discloses an active ingredient for use for the uses exemplified above. That is, the present invention discloses, for example, an active ingredient for use in culturing animal cells, active ingredient for use in improving growth of animal cells, active ingredient for use in promoting differentiation of animal cells, and active ingredient for use in manufacture of a composition for culturing animal cell.

### Examples

Hereafter, the present invention will be more specifically explained with reference to the following non-limiting examples.

### (1) Evaluation of activity of pea-based soy sauce (soy-free) using bovine muscle stem cells in serum-free medium.

### (1-1) Preparation of bovine muscle stem cells

Muscle tissues were collected from edible bovine shanks, and CD56-positive cells were isolated by using a magnetic activated cell separation system (MACS) and used as bovine muscle stem cells.

### (1-2) Evaluation of activity of pea-based soy sauce (soy-free) using bovine muscle stem cells in serum-free medium

The bovine muscle stem cells obtained in (1-1) were suspended in a serum medium. As the serum medium, Advanced DMEM (Thermo Fisher Scientific, Cat No. 12491015) supplemented with 20% FBS (Thermo Fisher Scientific, Cat No. 10270106), 10% horse serum (Thermo Fisher Scientific, Cat No. 16050-130), and 1% GlutaMAX^{™} Supplement (Thermo Fisher Scientific, Cat No. 35050061) was used. The cell suspension was inoculated onto a 6-well plate (BD FALCON, Cat # 353046) coated with 1 µg/cm² of fibronectin (Corning, Cat # F2006) at a cell density of 4200 to 5300 cells/2 mL/cm², and the medium was changed every other day. On day 4 from the start of the culture, the cells were subcultured to a serum-free medium or serum-free medium containing pea-based soy sauce (soy-free), and the medium was changed every other day. As the serum-free medium, StemFit^{R} Basic03 (Ajinomoto) supplemented with 5 ng/mL HGF (Peprotech, Cat# 294HGN005), 20 ng/mL EGF (Sigma Cat# E9644), and 10 ng/mL bFGF (Peprotech, Cat# AF-450-62) was used. As the serum-free medium containing pea-based soy sauce (soy-free), the serum-free medium supplemented with 0.33% pea-based soy sauce (soy-free) produced by San-J (product name, No Soy Gluten Free Tamari) or pea-based soy sauce (soy-free) produced by Kikkoman (product name, Itsudemo Shinsen Endomame Shoyu) was used. The group numbers and media are summarized in Table 1. The cells were counted and subcultured every 4 days, and the growth rate was calculated for two consecutive generations of subculture from day 4 to day 12.

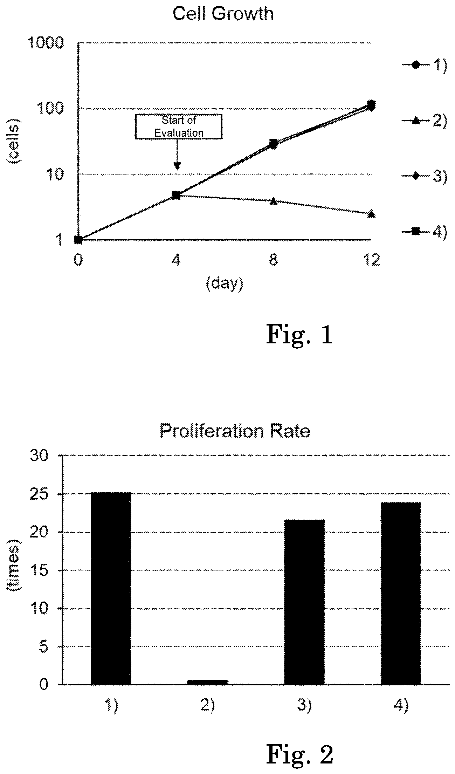

The results are shown in Figs. 1 and 2. Both the pea-based soy sauce (soy-free) of San-J and the pea-based soy sauce (soy-free) of Kikkoman promoted proliferation of the bovine muscle stem cells in the serum-free medium.

### [Table 1]

**Table 1**

| Group No. | Medium | Additive |
|---|---|---|
| 1) | Serum medium | No addition |
| 2) | Serum-free medium | No addition |
| 3) | Serum-free medium containing pea-based soy sauce (soy-free) | 0.33% Pea-based soy sauce (soy-free) (San-J) |
| 4) | Serum-free medium containing pea-based soy sauce (soy-free) | 0.33% Pea-based soy sauce (soy-free) (Kikkoman) |

### (1-3) Comparative evaluation of activities of pea-based soy sauce (soy-free), soy sauce and other extracts

The bovine muscle stem cells obtained in (1-1) were suspended in the serum medium. The cell suspension was inoculated onto a 6-well plate coated with 1 µg/cm² of fibronectin at a cell density of 5100 to 5300 cells/2 mL/cm², and the medium was changed every other day. On day 4 from the start of the culture, the cells were subcultured to a serum-free medium, serum-free medium containing pea-based soy sauce (soy-free), serum-free medium containing soy sauce, serum-free medium containing soy protein acid hydrolysate, or serum-free medium containing yeast extract, and the medium was changed every other day. The serum-free medium and the serum-free medium containing pea-based soy sauce (soy-free) are as described in (1-2). As the serum-free medium containing soy sauce, the serum-free medium supplemented with 0.38% of soy sauce (Kikkoman, product name: Itsudemo Shinsen Shiboritate Nama Shoyu Table-top bottle) was used. As the serum-free medium containing soy protein acid hydrolysate, the serum-free medium supplemented with soy protein acid hydrolysate (Sigma, Cat No. S1674) at a final concentration of 0.5 mg/mL was used. As the serum-free medium containing yeast extract, the serum-free medium supplemented with Bacto^{™} Yeast Extract (Thermo Fisher Scientific, Cat No. 212750) at a final concentration of 0.5 mg/mL was used. The group numbers and media are summarized in Table 2. The cells were counted and subcultured every 4 days, and proliferation rates were calculated for two consecutive generations of subculture from day 4 to day 12.

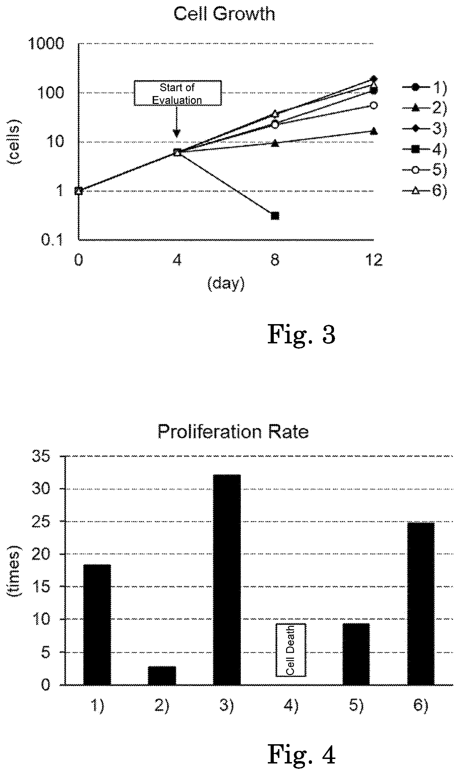

The results are shown in Figs. 3 and 4. The bovine muscle stem cells did not proliferate in the serum-free medium containing soy sauce. The soy protein acid hydrolysate partially promoted proliferation of the bovine muscle stem cells in the serum-free medium, but less effectively than the pea-based soy sauce (soy-free). The yeast extract promoted proliferation of the bovine muscle stem cells in the serum-free medium.

### [Table 2]

**Table 2**

| Group No. | Medium | Additive |
|---|---|---|
| 1) | Serum medium | No addition |
| 2) | Serum-free medium | No addition |
| 3) | Serum-free medium containing pea-based soy sauce (soy-free) | 0.33% Pea-based soy sauce (soy-free) (San-J) |
| 4) | Serum-free medium containing soy sauce | 0.38% Soy sauce |
| 5) | Serum-free medium containing soy protein acid hydrolysate | 0.5 mg/mL Soy protein acid hydrolysate |
| 6) | Serum-free medium containing yeast extract | 0.5 mg/mL Bacto^{™} Yeast Extract |

### (1-4) Comparative evaluation of activities of pea-based soy sauce (soy-free) and pea protein hydrolysate

The bovine muscle stem cells obtained in (1-1) were suspended in the serum medium. The cell suspension was inoculated onto a 6-well plate coated with 1 µg/cm² fibronectin at a cell density of 5300 cells/2 mL/cm², and the medium was changed every other day. On day 4 from the start of the culture, the cells were subcultured to a serum-free medium, serum-free medium containing pea-based soy sauce (soy-free), or serum-free medium containing pea protein hydrolysate, and the medium was changed every other day. The serum-free medium and the serum-free medium containing pea-based soy sauce (soy-free) were as described in (1-2). As the serum-free medium containing pea protein hydrolysate, the serum-free medium supplemented with Pea peptone (ORGANOTECHNIE) at a final concentration of 0.46 mg/mL was used. The group numbers and medium are summarized in Table 3. The cells were counted and subcultured every 4 days, and proliferation rates were calculated for two consecutive generations from day 4 to day 12.

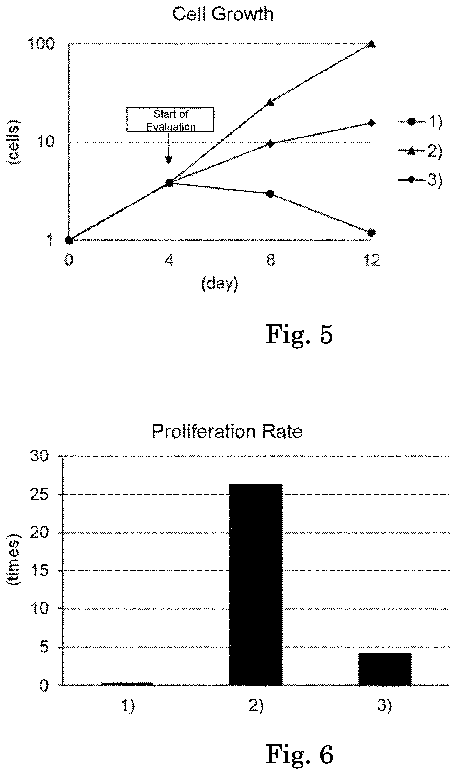

The results are shown in Figs. 5 and 6. The pea protein hydrolysate partially promoted proliferation of the bovine muscle stem cells in the serum-free medium, but was less effective than the pea-based soy sauce (soy-free).

### [Table 3]

**Table 3**

| Group No. | Medium | Additive |
|---|---|---|
| 1) | Serum-free medium | No addition |
| 2) | Serum-free medium containing pea-based soy sauce (soy-free) | 0.33% Pea-based soy sauce (soy-free) (San-J) |
| 3) | Serum-free medium containing pea protein hydrolysate | 0.46 mg/mL Pea peptone |

### (2) Evaluation of activity of pea protein using bovine muscle stem cells in serum-free medium

### (2-1) Preparation of bovine muscle stem cells

Muscle tissues were collected from edible bovine shanks, and CD56-positive cells were isolated by using a magnetic activated cell separation system (MACS) and used as bovine muscle stem cells.

### (2-2) Evaluation of activity of plant proteins using bovine muscle stem cells in serum-free medium

The bovine muscle stem cells obtained in (2-1) were suspended in a serum medium. As the serum medium, Advanced DMEM (Thermo Fisher Scientific, Cat No. 12491015) supplemented with 20% FBS (Thermo Fisher Scientific, Cat No. 10270106), 10% horse serum (Thermo Fisher Scientific, Cat No. 16050-130), and 1% GlutaMAX^{™} Supplement (Thermo Fisher Scientific, Cat No. 35050061) was used. The cell suspension was inoculated onto a 6-well plate (BD FALCON, Cat # 353046) coated with 1 µg/cm² of fibronectin (Corning, Cat # F2006) at a cell density of 4200 to 5300 cells/2 mL/cm², and the medium was changed every other day. On day 4 from the start of the culture, the cells were subcultured to a serum-free medium or serum-free medium containing pea protein, and the medium was changed every other day. As the serum-free medium, albumin-omitted StemFit^{R} Basic03 (Ajinomoto) supplemented with 5 ng/mL HGF (Peprotech, Cat# 294HGN005), 20 ng/mL EGF (Sigma Cat# E9644), 10 ng/mL bFGF (Peprotech, Cat# AF-450-62), and 0.33% pea-based soy sauce (soy-free) (San-J, product name: No Soy Gluten Free Tamari) was used. As the serum-free medium containing pea protein, the serum-free medium supplemented with 2.5 g/L pea protein (Roquette, product name: NUTRALYS S85F, protein content: approximately 85%) was used. Further, the following 7 types of plant proteins other than pea protein were also added to the serum-free medium at the concentrations shown in Table 4 to prepare serum-free media containing plant protein, and evaluated: wheat albumin (Nissin Pharma, product name: NA-1), barley/rice protein (Zea10, product name: Beretein^{™} Barley and Rice Protein), pumpkin seed protein (Shinhigh International Crops, product name: Organic Pumpkin Seed Protein), green gram protein (Organo Food Tech, product name: Orprotein^{R} MP-AC), mung bean protein (Shinhigh International Crops, product name: Mung Bean Protein), rapeseed protein (Merit Functional Foods, product name: Puratein^{R} C Canola Protein ), and hemp seed protein (Jinzhou Qiaopai Biotech, product name: Hemp Protein 60%). The group numbers and media are summarized in Table 4. The cells were counted and subcultured every 4 days, and the proliferation rates were calculated for two consecutive generations of subculture from day 4 to day 12.

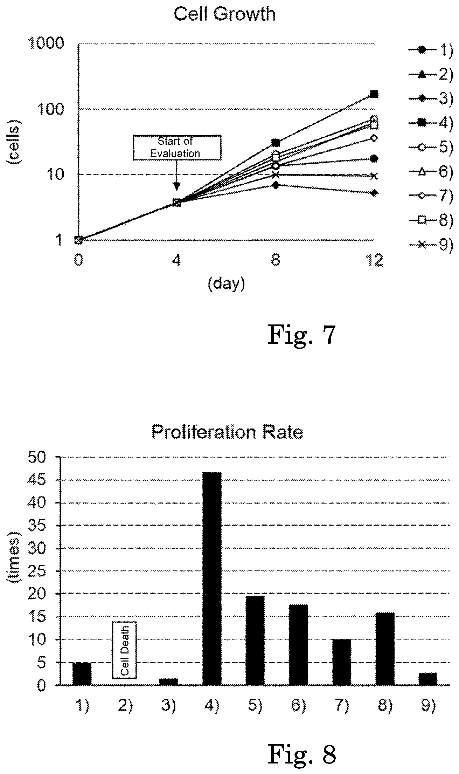

The results are shown in Figs. 7 and 8. Cell proliferation-promoting activities for the bovine muscle stem cells were observed for the pea protein, pumpkin seed protein, green gram protein, mung bean protein, and rapeseed protein, and in particular, the pea protein showed the highest activity.

### [Table 4]

**Table 4**

| Group No. | Additive |
|---|---|
| 1) | No addition |
| 2) | 2.5 g/L Wheat albumin |
| 3) | 2.5 g/L Barley/rice protein |
| 4) | 2.5 g/L Pea protein |
| 5) | 2.5 g/L Pumpkin seed protein |
| 6) | 2.5 g/L Green gram protein |
| 7) | 2.5 g/L Mung bean protein |
| 8) | 2.5 g/L Rapeseed protein |
| 9) | 2.5 g/L Hemp seed protein |

### (2-3) Evaluation of activity of soy protein using bovine muscle stem cells in serum-free medium

The bovine muscle stem cells obtained in (2-1) were suspended in a serum medium. The serum medium was as described in (2-2). The cell suspension was inoculated onto a 6-well plate coated with 1 µg/cm² of fibronectin at a cell density of 5300 cells/2 mL/cm², and the medium was changed every other day. On day 4 from the start of the culture, the cells were subcultured to a serum-free medium or serum-free medium containing soy protein, and the medium was changed every other day. As the serum-free medium, StemFit^{R} Basic03 (Ajinomoto) supplemented with 10 ng/mL bbFGF, 20 ng/mL EGF, 5 ng/mL HGF, and 0.5 g/L soy protein acid hydrolysate (Sigma, Cat No. S1674) was used. As the serum-free medium containing soy protein, the serum-free medium supplemented with 2.5 g/L soy protein (Japan Plant Protein Food Association, product name: Powdered Soy Protein) was used. The cells were counted and subcultured every 4 days, and the proliferation rate was calculated for a period of from day 4 to day 8.

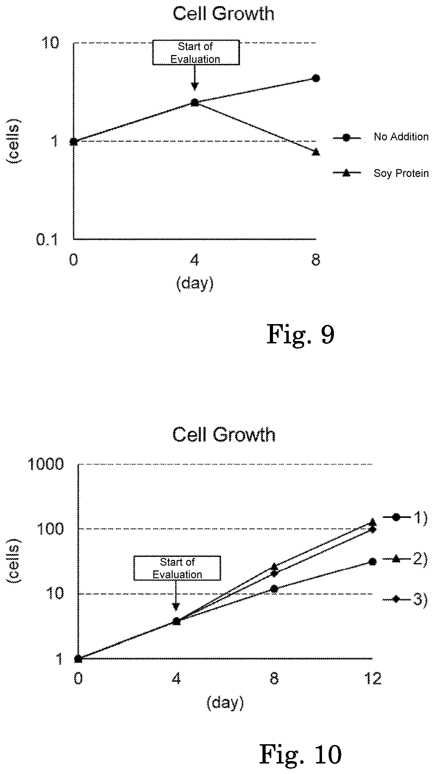

The results are shown in Fig. 9. The soy protein did not promote the proliferation of the bovine muscle stem cells in the serum-free medium.

### (2-3) Comparative evaluation of activities of different pea proteins

The bovine muscle stem cells obtained in (2-1) were suspended in a serum medium. The serum medium was as described in (2-2). The cell suspension was inoculated onto a 6-well plate coated with 1 µg/cm² of fibronectin at a cell density of 5300 cells/2 mL/cm², and the medium was changed every other day. On day 4 from the start of the culture, the cells were subcultured to a serum-free medium or serum-free medium containing pea protein, and the medium was changed every other day. As the serum-free medium, StemFit^{R} Basic03 (Ajinomoto) supplemented with 10 ng/mL bbFGF, 20 ng/mL EGF, 5 ng/mL HGF, and Bacto^{™} Yeast Extract (Thermo Fisher Scientific, Cat No. 212750) was used. As the serum-free medium containing pea protein, the serum-free medium supplemented with 2.5 g/L pea protein (Roquette, product name: NUTRALYS S85F, protein content: approximately 85%) or 2.5 g/L pea protein (HENGYUAN BIOTECHNOLOGY, product name: HYPP-A85%) was used. The group numbers and media are summarized in Table 5. The cells were counted and subcultured every 4 days, and proliferation rates were calculated for two consecutive generations from day 4 to day 12.

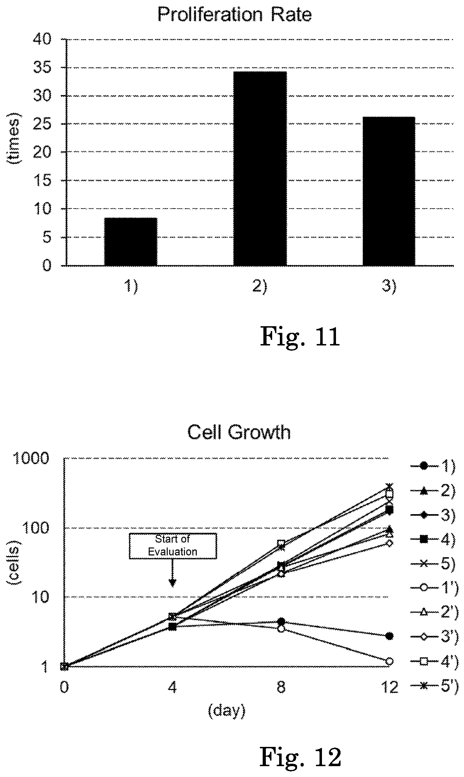

The results are shown in Figs. 10 and 11. Both the pea proteins promoted proliferation of the bovine muscle stem cells in the serum-free medium.

### [Table 5]

**Table 5**

| Group No. | Additive |
|---|---|
| 1) | No addition |
| 2) | 2.5 g/L Pea protein (NUTRALYS S85F) |
| 3) | 2.5 g/L Pea protein (HYPP-A85%) |

### (2-4) Evaluation of activity of combination of pea-based soy sauce (soy-free) and pea protein

The bovine muscle stem cells obtained in (2-1) were suspended in a serum medium. The serum medium was as described in (2-2). The cell suspension was inoculated onto a 6-well plate coated with 1 µg/cm² of fibronectin at a cell density of 4200 cells/2 mL/cm², and the medium was changed every other day. On day 4 from the start of the culture, the cells were subcultured to a serum-free medium or serum-free medium containing pea-derived product, and the medium was changed every other day. As the serum-free medium, StemFit^{R} Basic03 (Ajinomoto) supplemented with 10 ng/mL bbFGF, 20 ng/mL EGF, and 5 ng/mL HGF was used. As the serum-free medium containing pea-derived product, the serum-free medium supplemented with 0.33% pea-based soy sauce (soy-free), 2.5 g/L pea protein (Roquette, product name: NUTRALYS S85F, protein content: approximately 85%), 2.5 g/L pea protein (HENGYUAN BIOTECHNOLOGY, product name: HYPP-A85%), 0.33% pea-based soy sauce (soy-free) and 2.5 g/L pea protein, or 0.33% pea-based soy sauce (soy-free), 2.5 g/L pea proteins, and soy lecithin (Tsuji Oil, product name: SLP-White) was used. The group numbers and media are summarized in Table 6. The cells were counted and subcultured every 4 days, and proliferation rates were calculated for two consecutive generations from day 4 to day 12.

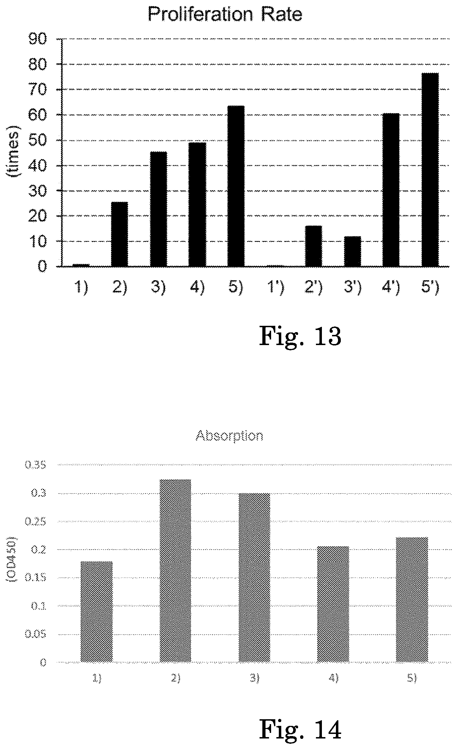

The results are shown in Figs. 12 and 13. The combinations of the pea-based soy sauce (soy-free) and pea proteins significantly promoted the cell proliferation. The combinations of the pea-based soy sauce (soy-free), pea proteins, and soy lecithin further promoted the cell proliferation.

**[Table 6]**

| Group No. | Medium | Additive |
|---|---|---|
| 1) | Serum-free medium | No addition |
| 2) | Serum-free medium containing pea-based soy sauce (soy-free) | 0.33% Pea-based soy sauce (soy-free) |
| 3) | Serum-free medium containing pea protein | 2.5 g/L Pea protein (NUTRALYS S85F) |
| 4) | Serum-free medium containing pea-based soy sauce (soy-free) and pea protein | 0.33% Pea-based soy sauce (soy-free) and 2.5 g/L pea protein (NUTRALYS S85F) |
| 5) | Serum-free medium containing pea-based soy sauce (soy-free), pea protein and soy lecithin | 0.33% Pea-based soy sauce (soy-free), 2.5 g/L pea protein (NUTRALYS S85F) and 10 mg/L soy lecithin |
| 1') | Serum-free medium | No addition |
| 2') | Serum-free medium containing pea-based soy sauce (soy-free) | 0.33% Pea-based soy sauce (soy-free) |
| 3') | Serum-free medium containing pea protein | 2.5 g/L Pea protein (HYPP-A85%) |
| 4') | Serum-free medium containing pea-based soy sauce (soy-free) and pea protein | 0.33% Pea-based soy sauce (soy-free) and 2.5 g/L pea protein (HYPP-A85%) |
| 5') | Serum-free medium containing pea-based soy sauce (soy-free), pea protein and soy lecithin | 0.33% Pea-based soy sauce (soy-free), 2.5 g/L pea protein (HYPP-A85%) and 10 mg/L soy lecithin |

### (3) Evaluation of activity of pea-based soy sauce (soy-free) using human muscle stem cells in serum-free medium

### (3-1) Preparation of human muscle stem cells

CD56-positive cells were isolated from human muscle stem cells derived from human fetuses (SCR, Cat No. 3510) using a magnetic activated cell separation system (MACS), and used as human muscle stem cells. The human muscle stem cells were expressing PAX7 and MyoD.

### (3-2) Evaluation of activity of pea-based soy sauce (soy-free) using human muscle stem cells in serum-free medium

The human muscle stem cells obtained in (3-1) were suspended in a serum medium. As the serum medium, DMEM (Wako, Cat No. 043-30085) supplemented with 10% FBS (Thermo Fisher Scientific, Cat No. 10099141) and 1% Chick Embryo Extract (United States Biological Cat No. C3999) was used. The cell suspension was inoculated onto a poly-L-lysine-coated 96-well plate (IWAKI, Cat No. 4020-040) at a density of 1 x 10³ cells/75 µL per well. After 2 hours, adhesion of the cells was confirmed, and then 75 µL of a serum-free medium not containing pea-based soy sauce (soy-free), serum-free medium containing pea-based soy sauce (soy-free) at a concentration 2-fold higher than the final concentration, or serum-free medium containing yeast extract was added. After 3 days of culture, Cell Count Reagent SF (Nacalai Tesque, Cat No. 07553-15) was added to each well, and the cells were cultured again for 2 hours in a CO₂ incubator. After the culture, the absorbance was measured at 450 nm by using a microplate reader to evaluate the cell proliferation ability. As the serum-free medium not containing pea-based soy sauce (soy-free), DMEM supplemented with 1% Chick Embryo Extract was used. As the serum-free medium containing pea-based soy sauce (soy-free), the serum-free medium not containing pea-based soy sauce (soy-free) supplemented with 0.1% or 0.33% of pea-based soy sauce (soy-free) (San-J, product name: No Soy Gluten Free Tamari) was used. As the serum-free medium containing yeast extract, the serum-free medium not containing pea-based soy sauce (soy-free) supplemented with Bacto^{™} Yeast Extract (Thermo Fisher Scientific, Cat No. 212750) at a final concentration of 0.2 mg/mL or 0.5 mg/mL was used. The group numbers and media are summarized in Table 7.

The results are shown in Fig. 14. Both the pea-based soy sauce (soy-free) and yeast extract promoted the proliferation of human muscle stem cells in the serum-free medium.

### [Table 7]

**Table 7**

| Group No. | Medium | Additive |
|---|---|---|
| 1) | Serum-free medium not containing pea-based soy sauce (soy-free) | No addition |
| 2) | Serum-free medium containing pea-based soy sauce (soy-free) 1 | 0.1% Pea-based soy sauce (soy-free) |
| 3) | Serum-free medium containing pea-based soy sauce (soy-free) 2 | 0.33% Pea-based soy sauce (soy-free) |
| 4) | Serum-free medium containing yeast extract 1 | 0.2 mg/mL Bacto^{™} Yeast Extract |
| 5) | Serum-free medium containing yeast extract 2 | 0.5 mg/mL Bacto^{™} Yeast Extract |

### (4) Evaluation of differentiation activity of pea-based soy sauce (soy-free) using chicken muscle stem cells in serum-free medium

### (4-1) Preparation of chicken muscle stem cells

Muscle stem cells were isolated from breast meat of edible chickens. The chicken muscle stem cells were expressing PAX7.

### (4-2) Evaluation of differentiation activity of pea-based soy sauce (soy-free) using chicken muscle stem cells in serum-free medium

The chicken muscle stem cells obtained in (4-1) were suspended in a serum medium. As the serum medium, DMEM (Nacalai Tesque, Cat No. 08456-65) supplemented with 10% chicken serum (Thermo Fisher Scientific, Cat No. 16110-082) and 5% bovine serum (Thermo Fisher Scientific, Cat No. 16050-130) was used. The cell suspension was inoculated onto a 96-well plate coated with 10 µg/cm² of gelatin (Sigma, Cat No. G1393-20ML) at a cell density of 60,000 cells, and the medium was replaced with a serum-containing differentiation medium or serum-free differentiation medium on the next day. As the serum-containing differentiation medium, DMEM/F12 (Thermo Fisher Scientific, Cat No. 08460-95) supplemented with 2% FBS (Thermo Fisher Scientific, Cat No. 26140-079) was used. As the serum-free differentiation medium, a serum-free differentiation medium not containing pea-based soy sauce (soy-free), serum-free differentiation medium containing pea-based soy sauce (soy-free), serum-free differentiation medium containing pea protein, serum-free differentiation medium containing soy sauce, serum-free differentiation medium containing soy protein acid hydrolysate, or serum-free differentiation medium containing yeast extract was used. As the serum-free differentiation medium not containing pea-based soy sauce (soy-free), DMEM/F12 supplemented with 1% NEAA (Thermo Fisher Scientific, Cat No. 11140050), 1 mM glutamine (Ajinomoto Healthy Supply), 73 mg/mL 0.2% selenium yeast (Medience), 30 ng/mL IGF-1 (Laurus Bio, Cat No. Rc LR3-IGF-1), and 10 µg/mL transferrin (Laurus Bio, Cat No. RTRF-P) was used. As the serum-free differentiation medium containing pea-based soy sauce (soy-free), serum-free differentiation medium containing pea protein, serum-free differentiation medium containing soy sauce, serum-free differentiation medium containing soy protein acid hydrolysate, and serum-free differentiation medium containing yeast extract, the serum-free differentiation medium not containing pea-based soy sauce (soy-free) supplemented with each of the additives shown in Table 8 was used. The group numbers and media are summarized in Table 8.

Then, the medium was changed every other day, and expression of the myosin heavy chain (MyHC) was measured by immunostaining on day 6. The cells were fixed with 4% paraformaldehyde (Nacalai Tesque, Cat No. 09154-85) for 15 minutes, washed twice with Dulbecco's phosphate buffered saline (Ca- and Mg-free, Nacalai Tesque, Cat. No. 14249-24), and immersed in Dulbecco's phosphate buffered saline (Ca- and Mg-free) containing 0.1% Triton X-100 (Sigma, Cat. No. T9284) for 10 minutes. Then, the buffer was replaced with a blocking buffer, and the cells were allowed to stand for 1 hour. As the blocking buffer, Dulbecco's phosphate buffered saline (Ca- and Mg-free) supplemented with 5% horse serum and 0.1% Triton X-100 was used. The buffer was then replaced with the blocking buffer containing 0.2% Anti-Myosin Heavy Chain Antibody, clone A4.1025 (Merck, Cat No. 05-716-I-25UL), and the cells were allowed to stand overnight at 4°C. On the next day, the cells were washed three times with Dulbecco's phosphate-buffered saline (Ca- and Mg-free), the buffer was replaced with the blocking buffer containing 0.1% Goat anti-Mouse IgG2a Antibody, Alexa Fluor 555 (Thermo Fisher Scientific, Cat No. A21137) and 0.1% Cellstain Hoechst 33342 solution (Dojin Chemical Laboratory, Cat No. H342), and the cells were allowed to stand for 1 hour at ordinary temperature. The cells were then washed three times with Dulbecco's phosphate buffered saline (Ca- and Mg-free) and the buffer was replaced with Dulbecco's phosphate buffered saline (Ca- and Mg-free). MyHC-positive cells were detected with a fluorescence microscope ECLIPSE Ti2-E (Nikon), and the fusion index (= Number of MyHC-positive nuclei/Number of total nuclei x 100 (%)) was calculated.

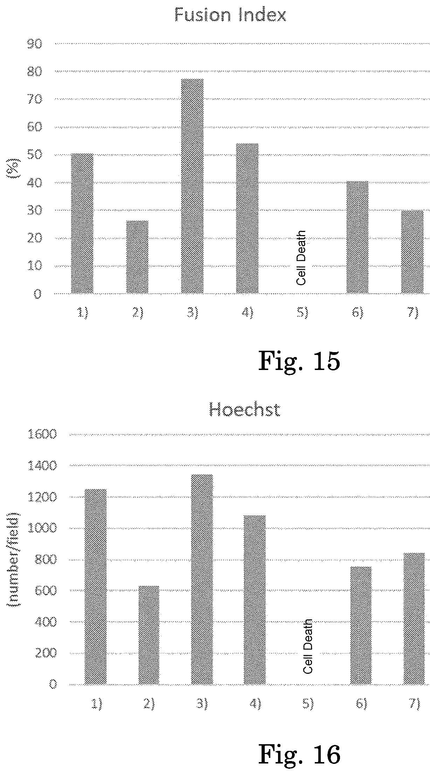

The results are shown in Figs. 15 and 16. The uses of the serum-free differentiation medium containing pea-based soy sauce (soy-free), serum-free differentiation medium containing pea protein, serum-free differentiation medium containing soy protein acid hydrolysate, and serum-free differentiation medium containing yeast extract more promoted myogenesis of the chicken muscle stem cells compared with the use of the serum-free differentiation medium not containing soy sauce (Fig. 15). In addition, the measurement results of the number of nuclei after muscle differentiation showed that the number of cells increased during the differentiation induction processes using these media (Fig. 16).

### [Table 8]

**Table 8**

| Group No. | Medium | Additive |
|---|---|---|
| 1) | Serum-free differentiation medium | No addition |
| 2) | Serum-free differentiation medium not containing pea-based soy sauce (soy-free) | No addition |
| 3) | Serum-free differentiation medium containing pea protein | 2.5 g/L Pea protein |
| 4) | Serum-free differentiation medium containing pea-based soy sauce (soy-free) | 0.33% Pea-based soy sauce (soy-free) |
| 5) | Serum-free differentiation medium containing soy sauce | 0.38% Soy sauce |
| 6) | Serum-free differentiation medium containing soy protein acid hydrolysate | 0.5 g/L Soy protein acid hydrolysate |
| 7) | Serum-free differentiation medium containing yeast extract | 0.5 g/L Bacto^{™} Yeast Extract |

### Industrial applicability

According to the present invention, animal cells can be cultured.

## Claims

1. A composition for culturing animal cells, which contains a pea-derived material and/or yeast extract.

2. The composition according to claim 1, which is for improving growth and/or promoting differentiation of animal cells.

3. The composition according to claim 1 or 2, which is a culture medium or a culture medium additive.

4. The composition according to claim 3, wherein the medium is a basal medium, a feed medium, or a perfusion medium.

5. The composition according to claim 1 or 2, wherein the pea-derived material is a pea fermentation product and/or a pea protein.

6. The composition according to claim 5, wherein the pea fermentation product is pea-based soy sauce (soy-free).

7. The composition according to claim 1 or 2, wherein the animal is a mammal, bird, fish, or crustacean.

8. The composition according to claim 1 or 2, wherein the animal is an animal for meat.

9. The composition according to claim 1 or 2, wherein the animal is a bovine or chicken.

10. The composition according to claim 1 or 2, wherein the cells are MyoD+ cells or PAX7+ cells.

11. The composition according to claim 1 or 2, wherein the cells are myoblasts or satellite cells.

12. The composition according to claim 1 or 2, which is substantially free of serum.

13. The composition according to claim 1 or 2, which is substantially free of animal-derived albumin.

14. A method for producing a product, which comprises
the step of culturing animal cells in the presence of a pea-derived material and/or yeast extract.

15. The method according to claim 14, wherein the product is cultured meat.

16. A method for culturing animal cells, which comprises
the step of culturing the animal cells in the presence of a pea-derived material and/or yeast extract.

17. A method for improving growth of animal cells, which comprises
the step of culturing the animal cells in the presence of a pea-derived material and/or yeast extract.

18. A method for promoting differentiation of animal cells, which comprises
the step of culturing the animal cells in the presence of a pea-derived material and/or yeast extract.

19. The method according to any one of claims 14 to 18, wherein the pea-derived material is a pea fermentation product and/or a pea protein.

20. The method according to claim 19, wherein the pea fermentation product is pea-based soy sauce (soy-free).

21. The method according to claim 19, wherein concentration of the pea fermentation product in a medium during the culture is 0.1 to 100 mg/mL.

22. The method according to claim 19, wherein concentration of the pea protein in a medium during the culture is 0.05 to 50 mg/mL.

23. The method according to any one of claims 14 to 18, wherein the animal is a mammal, bird, fish, or crustacean.

24. The method according to any one of claims 14 to 18, wherein the animal is an animal for meat.

25. The method according to any one of claims 14 to 18, wherein the animal is a bovine or chicken.

26. The method according to any one of claims 14 to 18, wherein the cells are MyoD+ cells or PAX7+ cells.

27. The method according to any one of claims 14 to 18, wherein the cells are myoblasts or satellite cells.

28. The method according to any one of claims 14 to 18, wherein the culture is performed in a medium substantially free of serum.

29. The method according to any one of claims 14 to 18, wherein the culture is performed in a medium substantially free of animal-derived albumin.
